# EUROPÄISCHE PATENTSCHRIFT

(11) **EP 2 919 888 B1**
(45) Veröffentlichungstag und Bekanntmachung des Hinweises auf die Patenterteilung: **17.07.2019**
(21) Anmeldenummer: 13774181.5
(22) Anmeldetag: 09.10.2013
(51) Int. Cl.: B01D 53/22, C10L 3/10

(54) **STEUERUNG DER GASZUSAMMENSETZUNG EINER GASSEPARATIONSANLAGE MIT MEMBRANEN**
CONTROL OF THE GAS COMPOSITION OF A GAS SEPARATING PLANT WITH MEMBRANES
CONTRÔLE DE LA COMPOSITION D'UN GAZ D'UNE INSTALLATION DE SÉPARATION DE GAZ PAR MEMBRANES

(30) Priorität: 14.11.2012 EP 12192571; 15.05.2013 EP 13167835
(43) Veröffentlichungstag der Anmeldung: 23.09.2015
(73) Patentinhaber: Evonik Fibres GmbH, 4861 Schörfling am Attersee (AT)
(72) Erfinder: UNGERANK, Markus, A-4320 Perg (AT); ROEGL, Harald, A-4702 Wallern an der Trattnach (AT)
(74) Vertreter: Evonik Patent Association
(86) Internationale Anmeldenummer: PCT/EP2013/071039
(87) Internationale Veröffentlichungsnummer: WO 2014/075850

(56) Entgegenhaltungen:
- EP-A1- 0 051 469
- WO-A1-2012/000727
- US-A- 4 806 132
- US-A- 5 053 058
- US-A- 5 281 253

## Beschreibung

Die vorliegende Erfindung betrifft ein Verfahren zur Steuerung einer Gasseparationsanlage, eine derart gesteuerte Anlage sowie deren Verwendung zur Separation von Gasgemischen, insbesondere bei der Aufbereitung von Bio- oder Erdgas.

Membranen sind bekannt dafür, dass mit ihrer Hilfe Gase relativ leicht voneinander in einem druckgetriebenen Prozess getrennt werden können. Die Gase werden dabei mit geringem Aufwand, aber meist auch mit geringer Reinheit der Produkte voneinander getrennt. Vor allem wenn bei einem Gasgemisch beide Komponenten einer binären Mischung möglichst rein isoliert werden sollen, bedarf es eines größeren Aufwandes bei der technischen Anordnung der Membranen und der Steuerung des Prozesses als bei einer simplen einstufigen Verschaltung, bei der beispielsweise nur die Retentatkomponente in bestimmter Reinheit hergestellt werden muss, das Permeat aber verworfen werden kann (z.B. Herstellung von Stickstoff aus Luft). Dieser vermehrte Aufwand besteht zum Beispiel bei der Trennung von Kohlendioxid und Methan (z.B. bei Erdgas oder Biogas), wo Methan erstens als Wertstoff möglichst in das Produktgas kommen soll, um so eine maximale Wertschöpfung zu erzielen und in möglichst geringer Konzentration im Abgas sein soll, da Methan ein klimaschädliches Gas ist und nicht in die Atmosphäre kommen soll. Auch bei der Auftrennung von Synthesegas in Kohlenmonoxid und Wasserstoff besteht eine ähnliche Aufgabenstellung.

Bei einer klassischen Trennung von einem binären Gasgemisch mit einer Membran ist die treibende Kraft der Trennung eine Partialdruckdifferenz der einzelnen Komponenten zwischen Retentat- und Permeatseite der Membrane. Dabei kann bei einem bestimmten Druckniveau auf der Retentatseite eine bestimmte Menge an Gasgemisch durch die Membran gefahren werden, um eine bestimmte Konzentration der langsameren Komponente im Retentatgas zu erhalten. Ändert sich nun die Zusammensetzung des Feedgases, so wird sich auch die Zusammensetzung des Retentatgases und des Permeatgases ändern. Die gleiche Änderung erfährt das System, wenn sich die Feedgasmenge ändert. Normalerweise werden die Konzentrationsänderungen in Retentatgas und oder Permeatgas als Steuergröße hergenommen und damit entweder die Feedgasmenge oder der Druck des Retentates so eingestellt, dass die gewünschte Konzentration in Permeat und/oder Retentat wieder hergestellt ist. Beispiele für derartige Steuerungen finden sich z. B. in der EP 1 324 815, der US 4,806,132 und der US 5,281,253.

Wie zuvor bereits erwähnt werden zur Isolierung möglichst reiner Endprodukte oft mehrstufige Membranverschaltungen verwendet. Beispiele hierfür sind in der WO 2012/000727, der US 6,565,626 und der US 6,168,649 zu finden.

Die Einstellung der Konzentrationen in den Produktströmen kann bei einer einstufigen oder retentatgestuft zweistufigen oder dreistufigen Verschaltung nicht ohne Beeinflussung der beiden Konzentrationen untereinander erfolgen. Ändert man zum Beispiel den Retentatdruck im Retentatproduktstrom (= Hauptdruck oder Betriebsdruck des Systems), so ändert sich nicht nur die Zusammensetzung des Retentates sondern auch die des Permeates. Analoges gilt bei Änderung der Feedgasmenge.

Die Verwendung einer dreistufigen Verschaltung wie sie bereits in der Anmeldung WO2012000727 A1 offenbart ist erlaubt es Methan bei der Auftrennung eines Gemisches von Methan und CO₂ mit einer Ausbeute von über 99% aufzureinigen, wobei die Reinheiten der Retentatgase und Permeatgase 97% deutlich übersteigen. Bei diesem Verfahren wird ein Gasgemisch aus mindestens zwei Komponenten in einer dreistufigen Verschaltung also so getrennt, dass zwei Komponenten relativ rein isoliert werden können, wenn es sich bei dem Gasgemisch um ein binäres Gemisch handelt. Ändert sich bei diesem Verfahren jedoch die Zusammensetzung des Rohgases oder ist eine größere oder kleinere Menge an Rohgas aufzubereiten, so ändern sich jeweils die Zusammensetzung des Retentatgases und des Permeatgases deutlich, was nicht gewünscht ist. Die Anbindung einer solchen Gasseparationsanlage an z. B. eine Biogasanlage ist daher problematisch. Steuert man nämlich Änderungen im Feedstrom durch eine Änderung des Hauptdruckes (= Betriebsdruck bzw. Druck im Retentatproduktstrom bzw. Retentatproduktgasdruck) entgegen, so ändert sich auch der Retentatvolumenstrom. Dies ist in vielen Fällen nicht erwünscht, da das Gas in diesen Fällen in eine Transportleitung eingespeist wird und diese einen Mindestdruck und oft auch ein Mindest- und/oder Maximalvolumen erfordert. Daher wird im Stand der Technik, z. B. der EP 1 324 815 teilweise vorgeschlagen in den Produktstrom einen weiteren Kompressor einzubauen, der den Druck für die Transportleitung regelt. Dies ist energetisch ungünstig, regelungstechnisch aufwendig und daher kommerziell wenig interessant. Weiterhin ist es bei einer dreistufigen Verschaltung - wie oben erläutert - mit der Einstellung des Hauptdruckes nicht möglich, die Permeatqualität unabhängig von der Retentatqualität zu beeinflussen.

Es besteht daher nach wie vor ein großer Bedarf an Anlagen zur Auftrennung von Gasgemischen bzw. Steuerung derselben, welche an Gasquellen mit wechselnder Rohgaszusammensetzung, -drücken und -mengen angeschlossen werden können und mindestens zwei Produkte in hoher Reinheit gleichzeitig, mit konstanter Qualität und konstantem Produktgasdruck liefern können.

Aufgabe der vorliegenden Erfindung war es daher ein Verfahren zur Steuerung einer Gasseparationsanlage bzw. eine derart gesteuerte Gasseparationsanlage zur Verfügung zu stellen, welche die Nachteile der Verfahren bzw. Anlagen des Standes der Technik nicht oder nur in verringertem Maße aufweisen.

Eine spezielle Aufgabe bestand darin ein Verfahren bzw. eine Anlage zur Verfügung zu stellen, welche(s) mindestens zwei Produkte in hoher Reinheit gleichzeitig liefern können. Eine ganz spezielle Aufgabe bestand darin, ein Verfahren bzw. eine Anlage zur Verfügung zu stellen, welche(s) mindestens zwei Produkte in hoher Reinheit gleichzeitig auch dann liefern können, wenn sich die Zusammensetzung und/oder der Druck und/oder die Menge des Rohgases ändern. Diese Anlage oder dieses Verfahren soll es insbesondere auch erlauben konstante Qualitäten, d.h. in engen Schwankungsbreiten, bevorzugt im kontinuierlichen Betrieb, zu liefern.

Das erfindungsgemäße Verfahren bzw. die Anlage soll in einer speziellen Aufgabe besonders flexibel sein und die Retentat- und Permeatqualitäten sollen unabhängig voneinander steuerbar sein. In einer ganz speziellen Aufgabe soll es möglich sein, die Kapazität der Anlage zu verändern, z. B. an Änderungen des Rohgasstromes anzupassen, ohne Membranflächen zu- oder abschalten zu müssen und/oder den Hauptdruck (Druck des Retentatproduktgasstroms) zu verändern, so dass Produktgasströme erhalten werden, die ohne zusätzliche Rekompression als, bevorzugt kontinuierlicher, Feed für eine Gastransportleitung geeignet sind.

Die erfindungsgemäße Steuerung soll vorzugsweise einfach und ggf. in bestehende Anlagen integrierbar sein.

Weitere nicht explizit genannte Aufgaben ergeben sich aus dem Gesamtzusammenhang der vorliegenden Beschreibung, Beispiele, Ansprüche und Zeichnungen.

Gelöst werden die erfindungsgemäßen Aufgaben durch eine Vorrichtung gemäß Anspruch 1 und 17 bzw. ein Verfahren nach Anspruch 8.

Das erfindungsgemäße Verfahren bzw. die erfindungsgemäße Vorrichtung sind dadurch gekennzeichnet, dass es sich um eine Membranverschaltung umfassend zumindest die Feedstromtrennstufe (1), die Retentattrennstufe (2) und die Permeattrennstufe (3)handelt, wobei der zweite Permeatstrom (9a + 9b) der Retentattrennstufe (2) und der dritte Retentatstrom (10a + 10b) der Permeattrennstufe (3) rückgeführt und mit dem Rohgasstrom gemischt werden. Durch die Steuerung der Flüsse der beiden Ströme (9a) und (10a) und damit des Permeatdrucks der Retentattrennstufe (2)und des Retentatdrucks der Permeattrennstufe (3) gelang es überraschender Weise die gestellten Aufgaben zu lösen. Es ist den Erfindern dadurch gelungen ein Verfahren bzw. eine Anlage bereitzustellen, bei denen die Reinheiten und Ausbeuten der Produktströme der Retentattrennstufe (2) und der Permeattrennstufe (3) unabhängig voneinander geregelt werden können. Es können sehr hohe Ausbeuten bei gleichzeitig sehr guten Reinheiten auch bei Schwankungen im Rohgasstrom erreicht werden.

Ferner ist es mit dem erfindungsgemäßen Verfahren möglich den Hauptdruck (Retentatdruck der Retentattrennstufe (2)) konstant zu halten, so dass die erfindungsgemäße Anlage ohne zusätzliche Kompressionseinrichtungen an eine Gastransportleitung angeschlossen werden kann.

Die erfindungsgemäße Anlage eignet sich besonders gut für die Aufbereitung von Rohgasströmen aus Biogasanlagen. In solchen Anlagen ändern sich die anfallende Menge Rohgas und die Zusammensetzung des Rohgases sehr oft. Die erfindungsgemäße Steuerung gleicht die Schwankungen problemlos aus.

Die erfindungsgemäße Regelung ist einfach und kann in bestehende Anlagen zur Gasseparation integriert werden.

Das erfindungsgemäße Verfahren ist auch dadurch flexibel, dass mehrere verschiedene Arten von Sensoren alleine oder gemeinsam verwendet werden können. Es können Zusammensetzungen von Strömen genauso zur Regelung der Drücke in den Trennstufen (2) und (3) verwendet werden wie Flüsse von Stoffströmen die diesen Trennstufen zugeführt werden. Insbesondere bei der Verwendung von Flusssensoren wird somit nach einer

Kalibrierung ein günstiges, schnelles, genaues und einfaches Verfahren zur Verfügung gestellt.

Gegenstand der vorliegenden Erfindung sind daher Vorrichtungen gemäß Anspruch 1 und 17 bzw. ein Verfahren nach Anspruch 8. Bevorzugte Ausführungsformen werden in den abhängigen Ansprüchen unter Schutz gestellt.

Die vorliegende Erfindung wird nachfolgend im Detail beschrieben. Zuvor werden einige wichtige Begriffe definiert.

Der Quotient der Permeanzen der Einzelgase ergibt die **Selektivität** der Membran zur Trennung bezüglich der zwei Gase und gibt somit an wie gut die Membran ein Gasgemisch bezüglich der beiden Komponenten auftrennen kann. Als **Permeat** wird der gesamte auf der Niederdruckseite der Membran, Membranmodule oder Membrantrennschritts anfallende Strom bezeichnet.

Als **Permeatgas** wird/werden die jeweils an der Membran, an dem Membranmodul, oder im Membrantrennschritt im **Permeatstrom** gegenüber den jeweiligen Eintrittsstrom angereicherten Komponente/Komponenten bezeichnet.

Als **Retentat** wird der gesamte auf der Hochdruckseite der Membran, Membranmodule oder Membrantrennschritts anfallende Strom bezeichnet, der nicht durch die Membran hindurch tritt.

Als **Retentatgas** wird/werden die jeweils an der Membran, an dem Membranmodul, oder im Membrantrennschritt im **Retentatstrom** gegenüber den jeweiligen Eintrittsstrom angereicherten Komponente/Komponenten bezeichnet.

**Rohgas** bzw. **Rohgasgemisch** bzw. **Rohgasstrom (17)** bezeichnet ein Gasgemisch aus mindestens zwei Gasen bzw. einen Strom dieses Gasgemisches, das/der mittels des erfindungsgemäßen Verfahrens bzw. der erfindungsgemäßen Vorrichtung aufgetrennt werden soll.

**Feedstrom (5)** bezeichnet einen Gasstrom, der der Feedstromtrennstufe (1) zugeführt wird. Dieser Strom kann zu Beginn der Durchführung des Verfahrens dem Rohgasstrom (17) bzw. dem durch einen Kompressor komprimierten Rohgasstrom entsprechen. Nach Rückführung des zweiten Permeatstroms (9b) bzw. des dritten Retentatstroms (10b) setzt sich der Feedstrom (5) aus den Gasen des Rohgasstrom (17), des zweiten Permeatstroms (9b) und des dritten Retentatstroms (10b) zusammen. Der Feedstrom (5) kann dabei erzeugt werden in dem die Ströme (9b) und (10b) entweder beide mit dem unkomprimierten Rohgasstrom (17) oder beide mit dem komprimierten Rohgasstrom oder einer mit dem unkomprimierten und einer mit dem komprimierten Rohgasstrom vermischt werden oder in dem die Ströme (9b) und/oder (10b) im Kompressor mit dem Rohgasstrom (17) vermischt werden. Kombinationen der zuvor beschriebenen Varianten sind von der vorliegenden Erfindung mit umfasst.

**Feedstromtrennstufe (1)** bezeichnet eine Membrantrennstufe zur Auftrennung des Feedstroms (5) in einen ersten Permeat- und einen ersten Retentatstrom (6) bzw. (7). **Retentattrennstufe (2)** bezeichnet eine Membrantrennstufe die gleich oder unterschiedlich zur Feedstromtrennstufe (1) aufgebaut sein kann, zur Auftrennung des ersten Retentatstroms (7) in einen zweiten Permeat- und einen zweiten Retentatstrom (9a + 9b) bzw. (8).

**Permeattrennstufe (3)** bezeichnet eine Membrantrennstufe die gleich oder unterschiedlich zur Feedstromtrennstufe (1) bzw. Retentattrennstufe (2) aufgebaut sein kann, zur Auftrennung des ersten Permeatstroms (6) in einen dritten Permeat- und einen dritten Retentatstrom (11) bzw. (10a + 10b).

Anhand der nachfolgend beschriebenen bevorzugten und speziellen Ausführungsformen des erfindungsgemäßen Verfahrens sowie der bevorzugten und besonders geeigneten Ausführungen sowie der Zeichnungen und Beschreibungen der Zeichnungen wird die Erfindung lediglich exemplarisch näher erläutert, d. h. sie ist nicht auf diese Ausführungs- und Anwendungsbeispiele oder auf die jeweiligen Merkmalskombinationen innerhalb einzelner Ausführungsbeispiele beschränkt.

Einzelne Merkmale, die im Zusammenhang mit konkreten Ausführungsbeispielen angegeben und/oder dargestellt sind, sind nicht auf diese Ausführungsbeispiele oder die Kombination mit den übrigen Merkmalen dieser Ausführungsbeispiele beschränkt, sondern können im Rahmen der technischen Möglichkeiten, mit jeglichen anderen Varianten, auch wenn sie in den vorliegenden Unterlagen nicht gesondert behandelt sind, kombiniert werden.

Gleiche Bezugszeichen in den einzelnen Figuren und Abbildungen der Zeichnungen bezeichnen gleiche oder ähnliche oder gleich oder ähnlich wirkende Komponenten. Anhand der Darstellungen in der Zeichnung werden auch solche Merkmale deutlich, die nicht mit Bezugszeichen versehen sind, unabhängig davon, ob solche Merkmale nachfolgend beschrieben sind oder nicht. Andererseits sind auch Merkmale, die in der vorliegenden Beschreibung enthalten, aber nicht in der Zeichnung sichtbar oder dargestellt sind, ohne weiteres für einen Fachmann verständlich.

Die vorliegende Erfindung betrifft eine Vorrichtung zur Trennung von Gasen umfassend als Membrantrennstufen zumindest die Feedstromtrennstufe (1), die Retentattrennstufe (2) und die Permeattrennstufe (3) sowie zumindest einen Kompressor (4) und/oder zumindest eine, bevorzugt eine oder zwei, Vakuumpumpe(n),
wobei
die Feedstromtrennstufe (1) einen Feedstrom (5), bestehend aus mindestens zwei Komponenten, in einen ersten Permeatstrom (6) und einen ersten Retentatstrom (7) auftrennt,
die Retentattrennstufe (2) den ersten Retentatstrom (7) in einen zweiten Permeatstrom (9a + 9b), wobei (9a) der Teilstrom vor der Steuereinrichtung (18) und nach der Retentattrennstufe (2) und (9b) den Teilstrom nach der Steuereinrichtung (18) kennzeichnet, und der Teilstrom (9b) dem Feedstrom (5) zugeführt wird, und einen zweiten Retentatstrom (8), der als Produkt entnommen oder weiterverarbeitet wird, aufteilt,
die Permeattrennstufe (3) den ersten Permeatstrom (6), in einen dritten Retentatstrom (10a + 10b), wobei (10a) der Teilstrom vor der Steuereinrichtung (19)und nach der Permeattrennstufe (3) und (10b) den Teilstrom nach der Steuereinrichtung (19) kennzeichnet, und der Teilstrom (10b) dem Feedstrom (5) zugeführt wird, und einen dritten Permeatstrom (11), der als Produkt entnommen oder weiterverarbeitet oder verworfen wird, aufteilt.

Die erfindungsgemäße Vorrichtung ist dadurch gekennzeichnet, dass
- der zweite Permeatstrom (9a + 9b) mindestens eine Permeatsteuereinrichtung (18) umfasst mit der Permeatdruck der Retentattrennstufe (2) erhöht oder erniedrigt werden kann und welche anhand von Messwerten eines oder mehrerer Messeinrichtungen (20a) im ersten Retentatstrom (7) und/oder eines oder mehrerer Messeinrichtungen (20b) im zweiten Retentatstrom (8) gesteuert wird und
- der dritte Retentatstrom (10a + 10b) mindestens eine Retentatsteuereinrichtung (19) umfasst mit der der Retentatdruck der Permeattrennstufe (3) erhöht oder erniedrigt werden kann und welche anhand von Messwerten eines oder mehrerer Messeinrichtungen (21a) im ersten Permeatstrom (6) und/oder eines oder mehrerer Messeinrichtungen (21b) im dritten Permeatstrom (11) gesteuert wird.

Das erfindungsgemäße Verfahren bzw. die erfindungsgemäße Vorrichtung zeichnet sich dadurch aus, dass es/sie derart ausgestaltet ist, dass auch bei schwankenden Zusammensetzungen oder Mengen oder Drücken des Rohgasstromes (17), welcher zusammen mit dem zweitem Permeatstrom (9b) und dem dritten Retentatstrom (10b) dem Feedstrom (5) zugeführt wird, durch die erfindungsgemäße Steuerung eine gleich bleibende Ausbeute und Qualität der beiden Produktgasströme (8) und (11) gewährleistet werden kann. Dabei ist besonders zu betonen dass die Reinheiten der Produktgasströme (8) und (11) unabhängig voneinander geregelt werden können, d.h. es gelang - im Gegensatz zu den Verfahren des Standes der Technik - die Regelung der Reinheit und Ausbeute beider Produktströme zu entkoppeln. Verantwortlich dafür sind die erfindungsgemäß verwendeten Steuereinrichtungen (18) und (19), welche in den Rückströmen (9a + 9b) und (10a + 10b) angeordnet sind.

Unter "Steuereinrichtungen" sind im Rahmen der vorliegenden Erfindung Geräte, Bauteile, Anlagen oder Teile von Anlagen zu verstehen, die eine Erhöhung oder Absenkung des Drucks in den Rückströmen (9a) und (10a) ermöglichen. Eine nicht abschließende Liste von möglichen Steuereinrichtungen umfasst: Druckminder- oder Druckerhöhungsventile, Gasentspannungseinrichtungen, Vakuumpumpen, Gebläse, Verdichter, insbesondere Kompressoren.

Die Steuereinrichtungen (18) und (19) werden anhand von Messwerten geregelt, welche durch die Messeinrichtungen (20a), (20b), (21a) und (21b) ermittelt werden. In einer ersten bevorzugten Ausführungsform der vorliegenden Erfindung ermitteln die Messeinrichtungen (20b) und (21b) als Parameter der Produktströme (8) und (11) den Gehalt einer oder mehrerer Komponenten in den Gasströmen. Die Ermittlung der Parameter mit den Messeinrichtungen (20b) und (21b) der Produktgasströme (8) und/oder (11) kann online oder offline erfolgen, je nach verwendeter Messeinrichtung. Bevorzugt erfolgt eine online Messung, weil damit die Regelung schneller erfolgen kann. Geeignete Messeinrichtungen sind dem Fachmann bekannt. Bevorzugt handelt es sich jedoch um Gasmessgeräte, die die Zusammensetzung der Gasströme für eine oder mehrere Komponenten messen können, insbesondere Inlinemessgeräte, die direkt im Gasstrom messen (z.B. mittels Infrarotabsorption oder Schallgeschwindigkeit, Dichte, Coreolis) und externe Messgeräte nach den gleichen Messprinzipien, die eine Probe aus dem Strom entnehmen und entweder kontinuierlich oder nicht kontinuierlich vermessen. Diese haben den Vorteil, dass die Zusammensetzung sehr schnell ermittelt werden kann und als Eingangsgröße in der Regelung sofort zur Verfügung steht

Kommt es zu einer Schwankung in der Zusammensetzung des Rohgases oder zu einer Veränderung der Menge oder des Drucks des Rohgasstroms (17) und/oder des Feedstroms (5), so ändern sich ohne Gegensteuerung auch die Eigenschaften, z. B. die Zusammensetzungen, der Produktströme (8) und (11). Durch die Messeinrichtungen (20b) und (21b) werden entsprechende Veränderungen registriert und eine Gegensteuerung mit Hilfe der Steuereinrichtungen (18) und (19) eingeleitet, so dass die erfindungsgemäße Anlage derart geregelt werden kann, dass die Eigenschaften, insbesondere die Zusammensetzungen, der Produktgasströme (8) und (11) wieder in einem vorgegebenen Bereich/Korridor liegen. Die erfindungsgemäße Anlage erlaubt es dabei beide Produktgasströme (8) und (11) gleichzeitig zu regeln aber auch nur einen von beiden Strömen im vorgegebenen Korridor zu halten.

In dieser ersten bevorzugten Ausführungsform der vorliegenden Erfindung sind daher Verfahren Gegenstand der Erfindung, bei denen die Steuerung der erfindungsgemäßen Anlage nach den folgenden Alternativen erfolgt:
i. Die Konzentration einer schwerer permeierenden Komponente B, ggf. auch ermittelt durch einen dazu korrelierenden Parameter, des zweiten Retentatstroms (8) fällt unter einen vorgegebenen Sollwert, der Druck des zweiten Permeatstroms (9a) wird daher mittels der Permeatsteuereinrichtung (18) erniedrigt, bis besagter Parameter, insbesondere die gewünschte Konzentration, wieder im Sollbereich liegt,
ii. Die Konzentration einer schwerer permeierenden Komponente B, ggf. auch ermittelt durch einen dazu korrelierenden Parameter, des zweiten Retentatstroms (8) steigt über einen vorgegebenen Sollwert, der Druck des zweiten Permeatstroms (9a) wird daher mittels der Permeatsteuereinrichtung (18)erhöht, bis besagter Parameter, insbesondere die gewünschte Konzentration, wieder im Sollbereich liegt.
iii. Die Konzentration einer schwerer permeierenden Komponente B, ggf. auch ermittelt durch einen dazu korrelierenden Parameter, des dritten Permeatstroms (11) fällt unter einen vorgegebenen Sollwert, der Druck des dritten Retentatstroms (10a) wird daher mittels der Retentatsteuereinrichtung (19) erhöht, bis besagter Parameter, bevorzugt die gewünschte Konzentration, wieder im Sollbereich liegt.
iv. Die Konzentration einer schwerer permeierenden Komponente B, ggf. auch ermittelt durch einen dazu korrelierenden Parameter, des dritten Permeatstroms (11) steigt über einen vorgegebenen Sollwert, der Druck des dritten Retentatstroms (10a) wird daher mittels der Retentatsteuereinrichtung (19) erniedrigt, bis der Parameter, insbesondere die gewünschte Konzentration, wieder im Sollbereich liegt.

In einer zweiten bevorzugten Ausführungsform umfasst die erfindungsgemäße Vorrichtung die Messeinrichtungen (20a) und (21a). Die Messeinrichtungen (20a) und (21a) ermitteln Parameter des ersten Retentatstroms (7) bzw. des ersten Permeatstroms (6) wie z. B. den Volumenstrom. In dieser Ausführungsform werden also nicht die Eigenschaften der

Produktströme (8) und (11) analysiert, sondern Eigenschaften der Gasströme, welche der zweiten bzw. der dritten Membrantrennstufe zugeführt werden.

Kommt es zu einer Schwankung in der Zusammensetzung oder zu einer Veränderung der Menge oder des Drucks des Rohgasstromes (17) oder des Feedstroms (5), so wirkt sich dies, ohne Gegensteuerung, auf die Eigenschaften, z. B. die Zusammensetzung bzw. die Mengen und die Drücke, des ersten Permeatstroms (6) bzw. des ersten Retentatstroms (7) aus. Die Messeinrichtungen (20a) und (21a) registrieren entsprechende Veränderungen. Durch eine Kalibrierung der Anlage können diese Eigenschaften des ersten Permeatstroms (6) mit denen des dritten Permeatstroms (11) (zweiter Produktstrom) und die des ersten Retentatstroms (7) mit denen des zweiten Retentatstroms (8) (erster Produktstrom) korreliert werden. Somit können auch mit den Messeinrichtungen (20a) und (21a) die Eigenschaften, insbesondere die Zusammensetzung und Ausbeute, der beiden Produktströme (8) und (11) geregelt werden. Dies erfolgt erneut mit Hilfe der Steuereinrichtungen (18) und (19). Die Steuerung der Zusammensetzungen der Produktgasströme (8) und (11) ist auch in dieser Ausführungsform entkoppelt und kann unabhängig voneinander geregelt werden. Unter Eigenschaften des zweiten Retentatstroms (8) (erster Produktstrom) und des dritten Permeatstroms (11) (zweiter Produktstrom) werden in diesem Fall Parameter verstanden, die am jeweiligen Strom gemessen werden können und welche durch die erfindungsgemäße Anlage in einem bestimmten Bereich gehalten oder hineingebracht werden sollen. Besonders bevorzugt handelt es sich um die Zusammensetzung und oder den Druck und oder die Menge bzw. den Volumenstrom der jeweiligen Produktströme, da diese Parameter für die Einspeisung des Produktgases in eine Pipeline in bestimmten Grenzbereichen liegen müssen. Diese Eigenschaften bzw. parameter werden im Rahmen der vorliegenden Erfindung auch als mit dem jeweiligen Volumenstrom des ersten Retentatstroms (7) oder des ersten Permeatstroms (6) korrelierte Eigenschaften bezeichnet,

Wie bereits erläutert muss in dieser Ausführungsform der vorliegenden Erfindung zunächst einmalig eine Kalibrierung der Anlage erfolgen. Dieser anfängliche Mehraufwand wird jedoch dadurch überkompensiert, dass nach der Kalibrierung eine einfache Flussmessung der Ströme (6) und (7) erfolgen kann, was schneller und billiger ist als z. B. kontinuierlich die Zusammensetzung der Produktgasströme (8) und (11) zu kontrollieren.

Nachfolgend werden die Grundprinzipien der Kalibrierung am Beispiel einer Biogasanlage mit einer erfindungsgemäßen dreistufigen Verschaltung erläutert. Die Kalibrierung der erfindungsgemäßen Vorrichtung kann wie folgt erfolgen:
Man legt zuerst die Sollkonzentrationen der langsamer permeierenden Komponente B im dritten Permeatstrom (11) und im zweiten Retentatstrom (8) fest. Anschließend variiert man z. B. die Zusammensetzung des Rohgasstroms (17) und bestimmt mit Hilfe der Messeinrichtungen (20a) und (21a) die Veränderungen der Zielparameter, in dieser Beispielkalibrierung des Volumenstroms, des ersten Retentatstroms (7) und des ersten Permeatstroms (8). Parallel bestimmt man mit Hilfe der Messeinrichtungen (20b) und (21b), z. B. mittels Gassensoren, die Veränderungen der Zusammensetzungen des dritten

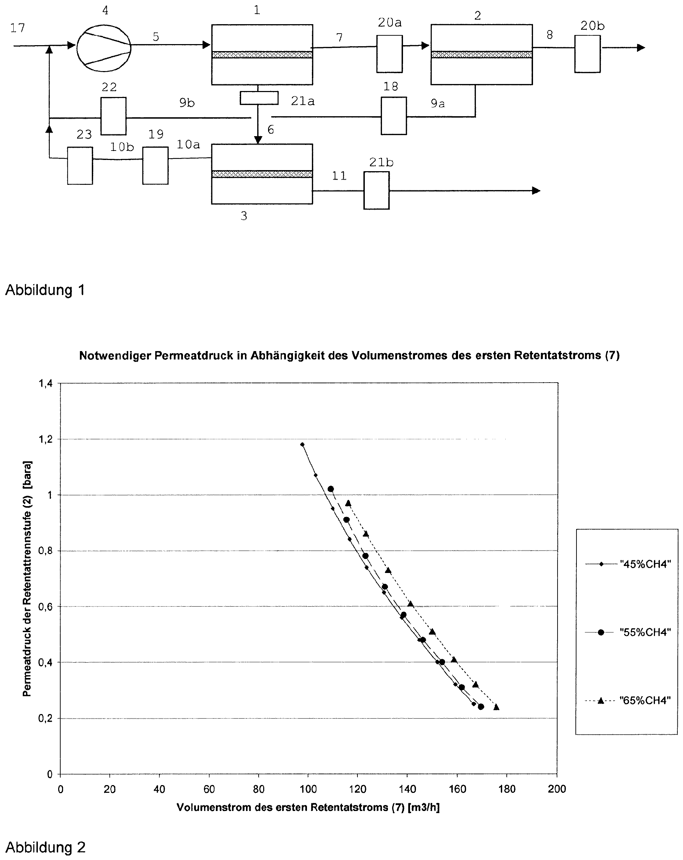

Permeatstroms (11) und des zweiten Retentatstroms (8). Zusätzlich misst man den Permeatdruck der Retentattrennstufe (2) und den Retentatdruck der Permeattrennstufe (3). Mit Hilfe der Steuereinrichtungen (18) bzw. (19) kann man besagte Permeat- bzw. Retentatdrücke verändern bis die Sollkonzentration der Komponente B im dritten Permeatstrom (11) und im zweiten Retentatstrom (8) wieder erreicht ist. Man kann nun die gemessenen Volumenströme des ersten Retentatstroms (7) gegen den Permeatdruck der Retentattrennstufe (2) und die Volumenströme des ersten Permeatstroms (6) gegen den Retentatdruck der Permeattrennstufe (3) auftragen. In Abbildung 2 ist beispielhaft zu sehen, wie der Druck im Permeat der Retentattrennstufe (2) eingestellt werden muss, um die Konzentration der der schwerer permeierenden Komponente B im Retentatstrom (8) konstant zu halten. Es ist zu erwähnen, dass die Gaszusammensetzung des Rohgasstromes hier eine Parallelverschiebung der Kurven bewirkt. Dies ist auch in Abb. 2 zu sehen, wo der Kurvenverlauf des notwendigen Permeatdruckes der Retentattrennstufe (2) in Abhängigkeit des Volumenstromes des ersten Retentatstroms (7) für drei unterschiedliche Rohgaszusammensetzung (45, 55 und 65% der Komponente B) angegeben ist. Wie man sieht ergibt sich für jede Rohgaszusammensetzung eine eigene separate Kurve.

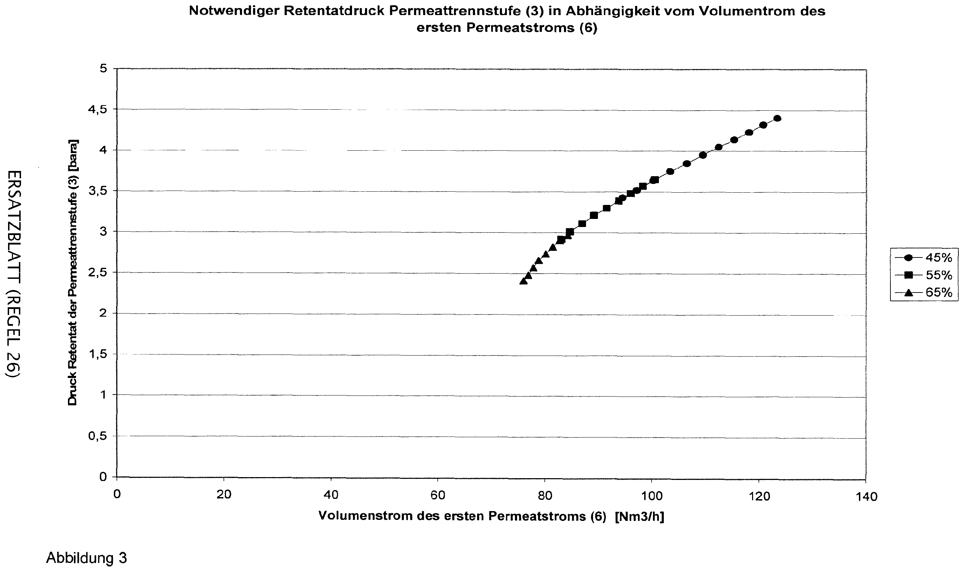

Ist die Zielsetzung der Trennung lediglich, eine Mindestqualität der schlechter permeierenden Komponente B im zweiten Retentatstrom (8) sicherzustellen, so kann zur Vereinfachung des erfindungsgemäßen Verfahrens auf die Ermittlung verschiedener Kurven für unterschiedliche Konzentration der schlechter Permeierenden des Rohgases verzichtet werden und nur mit der Arbeitskurve mit der höchsten Konzentration an leichter permeabler Komponente A gearbeitet werden. Alternativ kann nur mit der Arbeitskurve mit der niedrigsten Konzentration an schlechter permeabler Komponente B gearbeitet werden. Erhöht sich die Konzentration der schlechter permeierenden Komponente B im Rohgas, so müsste eigentlich der Druck im Permeat der Stufe 2 erhöht werden, um die Konzentration der schlechter permeierenden Komponente B im Retentatgas der Stufe 2 konstant zu halten. Wird der Druck nicht angepasst, so steigt die Konzentration an Komponente B im Retentat der Stufe 2, sie liegt jedoch unter Verwendung der Kalibriergeraden mit der niedrigsten Konzentration an Komponente B im Rohgas immer über dem definierten Mindestsollwert. Während sich beim Retentatstrom (8) mehrere Kurven bei unterschiedlichen Rohgaszusammensetzungen ergeben, so können die Daten bei den Drücken des Retentates der Permeattrennstufe (3) in Abhängigkeit des Volumenstromes des ersten Permeatstroms (6) auf einer Kurve liegen (siehe hierzu als Beispiel Abbildung 3).

Unter Verwendung der für die erhaltenen Kurven abgeleiteten mathematischen Funktionen ist es nun alleine durch Messung der Volumenströme mittels der Messeinrichtungen (20a) und/oder (21a) möglich, auch ohne aufwändigere Messung von Konzentrationen in den Produktströmen mittels der Messeinrichtungen (20b) und (21b), eine schnelle Steuerung der Anlage zu gewährleisten, auch wenn sich die Rohgaszusammensetzung ändert oder wenn mehr Rohgas aufbereitet werden soll.

Zur Aufbereitung einer sich variierenden Menge Rohgas ist es vorteilhaft, wenn die Steuerung (Kontrolleinrichtung) des Kompressors ein Signal eines Füllstandsmessers der Biogasanlage (z.B. Gassack oder Druck im Fermenter) oder eines Sensors im Rohgasstrom (17) bekommt. Der Kompressor kann dann auch so gesteuert werden, dass das Sollniveau an Rohbiogas erhalten bleibt. Die Anlage regelt sich dann selber nach oben beschriebenem Regelmechanismus. Details zu dieser bevorzugten Ausführungsform finden sich weiter unten.

Als Messeinrichtungen (20a) und/oder (21a) werden Durchflussmesser (Masse oder Volumen) verwendet. Die Bestimmung der Parameter mittels der Messeinrichtungen (20a) und (21a) kann online oder offline erfolgen. Bevorzugt erfolgt eine online Messung. Geeignete Messeinrichtungen sind dem Fachmann bekannt.

Gegenstand der vorliegenden Erfindung in dieser Ausführungsform sind daher Verfahren, bei denen die Steuerung der erfindungsgemäßen Anlage bevorzugt nach einer oder mehreren der folgenden Alternativen erfolgt:
v. Der Volumenstrom des ersten Retentatstroms (7), ggf. auch ermittelt durch einen dazu korrelierenden Parameter, steigt über einen vorgegebenen Sollwert, der Druck des zweiten Permeatstroms (9a) wird daher mittels der Permeatsteuereinrichtung (18) soweit erniedrigt, bis gemäß der Kalibrierkurve der notwendige Druck erreicht ist und somit die gewünschte Eigenschaft des zweiten Retentatstroms (8), bevorzugt die Zusammensetzung des zweiten Retentatstroms (8), wieder im Sollbereich liegt,
vi. Der Volumenstrom des ersten Retentatstroms (7), ggf. auch ermittelt durch einen dazu korrelierenden Parameter, sinkt unter einen vorgegebenen Sollwert, der Druck des zweiten Permeatstroms (9a) wird daher mittels der Permeatsteuereinrichtung (18)soweit erhöht, bis der notwendige Druck anhand der Kalibrierkurve erreicht ist und somit die gewünschte Eigenschaft des zweiten Retentatstroms (8), bevorzugt die Zusammensetzung des zweiten Retentatstroms (8), wieder im Sollbereich liegt,
vii. Der Volumenstrom des ersten Permeatstroms (6), ggf. auch ermittelt durch einen dazu korrelierenden Parameter, steigt über einen vorgegebenen Sollwert, der Druck des dritten Retentatstroms (10a) wird daher mittels der Retentatsteuereinrichtung (19) soweit erhöht, bis der notwendige Druck anhand der Kalibrierkurve erreicht ist und somit die gewünschte Eigenschaft des dritten Permeatstroms (11), bevorzugt die Zusammensetzung des dritten Permeatstroms (11), wieder im Sollbereich liegt,
viii. Der Volumenstrom des ersten Permeatstroms (6), ggf. auch ermittelt durch einen dazu korrelierenden Parameter, sinkt unter einen vorgegebenen Sollwert, der Druck des dritten Retentatstroms (10a) wird daher mittels der Retentatsteuereinrichtung (19) soweit erniedrigt, bis der notwendige Druck anhand der Kalibrierkurve erreicht ist und somit die gewünschte Eigenschaft des dritten Permeatstroms (11), bevorzugt die Zusammensetzung des dritten Permeatstroms (11), wieder im Sollbereich liegt.

Ein großer Vorteil der erfindungsgemäßen Anlage und des erfindungsgemäßen Verfahrens liegt in der höchst variablen Anlagenkapazität, d.h. der Möglichkeit die Anlagenleistung variabel steuern und an den Bedarf an Produktgas anpassen zu können. Dies kann wie bereits erwähnt ohne Zu- bzw. Abschaltung von Membranflächen erfolgen. In einer bevorzugten Ausführungsform der vorliegenden Erfindung wird dazu die Anlagenleistung der erfindungsgemäßen Anlage durch eine Änderung des Fördervolumens des Kompressors (4) erhöht oder erniedrigt und einer dadurch verursachten Änderung der Konzentration der schwerer permeierenden Komponente B im zweiten Retentatstrom (8) gemäß den Verfahrensalternativen i bzw. ii und einer dadurch verursachten Änderung der Konzentration der schwerer permeierenden Komponente B im dritten Permeatstrom (11) gemäß den Verfahrensalternativen iii bzw. iv und/oder einer dadurch verursachten Änderung des Flusses des ersten Retentatstroms (7) gemäß den Verfahrensalternativen v bzw. vi und/oder einer dadurch verursachten Änderung des Flusses des ersten Permeatstroms (6) gemäß den Verfahrensalternativen vii bzw. viii entgegengewirkt.

Die zuvor beschriebenen Verfahren i bis viii können miteinander kombiniert bzw. Mischformen angewendet werden. Unter Messeinrichtung (20a), (20b), (21a) oder (21b) werden einzelne Messgeräte, Apparaturen etc. aber auch Kombinationen oder Zusammenschaltungen mehrerer Geräte, Apparaturen etc. verstanden.
Die Messeinrichtungen (20a), (20b), (21a) oder (21b) können untereinander in den verschiedenen Verfahrensalternativen flexibel kombiniert werden. So kann z.B. eine Messeinrichtung (20a) zusammen mit einer Messeinrichtung (20b) verwendet werden um die Permeatsteuereinrichtung (18)zu regeln. In diesem Fall hätte man ein Backup Messsystem, welches ermöglicht die Messsysteme gegenseitig zu kontrollieren und abzusichern. Somit kann z. B. festgestellt werden, wenn eine Messeinrichtung kaputt ist. Entsprechende Ausgestaltungsformen der vorliegenden Erfindung sind für einen Fachmann anhand der Beschreibung und den Beispielen der vorliegenden Erfindung leicht auffindbar und von der Erfindung mit erfasst.

Je nach verwendeter Mess- und/oder Steuereinrichtung sowie deren Anzahl kann es vorteilhaft sein zwischen die Mess- und die Steuereinrichtung mindestens eine Datenverarbeitungseinrichtung (in den Abbildungen nicht gezeigt), bevorzugt mindestens einen Computer, zwischenzuschalten. Dadurch kann die erfindungsgemäße Vorrichtung bzw. das erfindungsgemäße Verfahren leicht zentral geregelt und die verschiedenen Messwerte bzw. Regelungsschritte protokolliert und koordiniert werden. Entsprechende technische Lösungen sind auf dem Markt erhältlich bzw. dem Fachmann bekannt und vom Umfang der vorliegenden Erfindung mit umfasst.

Im erfindungsgemäßen Verfahren werden im ersten Retentatstrom (7) und/oder im ersten Permeatstrom (6) Durchflussmesser als Messeinrichtung (20a) und/oder (21a) verwendet.

Bevorzugt werden im zweiten Retentatstrom (8) und/oder im dritten Permeatstrom (11) eine online oder offline Messeinrichtung (20b) und/oder (21b) zur Bestimmung der Zusammensetzung des jeweiligen Gasgemisches verwendet.
Neben der Steuerung der Stoffströme (9a + 9b) und (10a + 10b) durch die Steuereinrichtungen (18) und (19), wie zuvor beschrieben, umfasst die vorliegende Erfindung auch Ausführungsformen, in denen noch weitere Steuerungen bzw. Regelungen in der Vorrichtung bzw. im Verfahren vorgenommen werden.

In einer weiteren bevorzugten Ausführungsform umfasst die erfindungsgemäße Vorrichtung eine Kontrolleinrichtung (24) (in den Abbildungen nicht gezeigt), welche die Leistung des Kompressors (4), bevorzugt dessen Drehzahl und damit dessen Fördervolumen, regelt. Ein Beispiel hierfür wäre ein Frequenzumformer.

Mittels der Kontrolleinrichtung wird bevorzugt die Leistung des Kompressors an die zu trennende Menge Rohgas (z.B. die Produktion von Biogas im Fermenter) oder an die Menge zu produzierende Menge Produktgas (z.B. Methanstrom im Retentatstrom (8)) anpasst. Die Änderung der Produktionsmenge an Rohgas (z.B. Rohbiogas aus einer Biogasanlage) kann zum Beispiel an einer Füllstandsanzeige eines Rohgaszwischenspeichers oder am Druck des Rohgases in einem Fermenter abgelesen werden. Steigt der Füllstand oder der Druck im Fermenter, so kann die Trennkapazität der Membrantrennanlage durch Erhöhung der Drehzahl des Verdichters erhöht werden. Somit kann der Druck im Fermenter oder der Füllstand im Zwischenspeicher konstant gehalten oder gesenkt werden. Sinkt im Gegensatz dazu der Füllstand im Zwischenspeicher oder der Druck im Fermenter so kann durch Reduktion der Drehzahl des Kompressors die Trennkapazität der Membrantrennanlage verringert werden und somit der Füllstand im Zwischenspeicher oder der Druck im Fermenter konstant gehalten oder verringert werden. Bei Veränderung des Feedvolumenstromes (5), die sich durch eine Änderung der Drehzahl des Kompressors ergibt, würde sich Änderung der Zusammensetzung des Retentatstromes (8) und des Permeatstroms (11) ergeben. Ein großer Vorteil der vorliegenden Erfindung liegt darin, dass diese Veränderung durch in dieser Erfindung weiter oben beschriebenen Regelmechanismen verhindert werden kann. Mit dieser erfindungsgemäßen Steuerung der Anlage für die Kapazität und die Qualität der Produktgase ist es nun möglich, die Trennkapazität der Anlage und die Zusammensetzung der Produktgasströme (8) und (11) unabhängig von einander zu steuern. Man kann daher die Kapazität der Anlage in einem bestimmten Rahmen unter Zuhilfenahme der Steuereinrichtungen (18) und (19) mit Hilfe der Kontrolleinrichtung des Kompressors verändern, ohne dass sich die Produktgasqualitäten in den Strömen (8) und (11) ändern, ohne dass der Retentatdruck in den Trennstufen (1) und (2) angepasst werden müsste und ohne dass Membranflächen zu- oder weggeschaltet werden müsste.

Diese Flexibilität der Kapazität kann auf einen bestimmten Bereich der Kompressordrehzahl und damit Rohgasfördervolumen beschränkt sein, welche vom Design der Anlage bestimmt wird, insbesondere hinsichtlich Druck in den Trennstufen, Flächenverhältnissen der Membranen in den einzelnen Trennstufen (1), (2) und (3) und vor allem hinsichtlich der

Steuereinrichtungen (18) und (19) und deren Bandbreite bei der Einstellung des Druckes in den jeweiligen Gaströmen (9a) und (10a). Wenn beispielsweise der minimale mögliche Druck im Permeatstrom (9a) 0,3 bara beträgt, so ist dies der limitierende Parameter für den Feedgasvolumenstrom (5). Eine weitere Erhöhung des Feedgasvolumenstroms (5) würde bedeuten, dass die Konzentration der langsameren Komponente B im Retentatstrom (8) unter den Sollwert sinken würde und somit der gewünschte Betriebspunkt der Anlage nicht mehr eingehalten werden könnte. Das gleiche gilt für den Fall, dass der Druck im Permeat (9a) nicht mehr weiter erhöht werden könnte als zum Beispiel Umgebungsdruck. Dieser Druck limitiert nun die Absenkung des Feedvolumenstromes (5), da bei weiterer Absenkung Feedvolumenstromes (5)der Gehalt an schwerer permeierender Komponente B im Retentat (8) über den Sollwert steigen würde.

Unter "Kontrolleinrichtung" wird hierbei die Steuereinheit des Kompressors verstanden, welche die Leistung des Kompressors, bevorzugt dessen Drehzahl regelt. Diese Kontrolleinrichtung kann derart ausgestaltet sein, dass sie Messdaten von Sensoren im Rohgasstrom (17) und/oder in vorgelagerten Speichern oder Produktionseinrichtungen verarbeitet. Beispielsweise kann das Signal der Messeinrichtung dann einen Frequenzumformer eines Verdichters steuern. Entsprechende Kompressoren und Kontroll- bzw. Steuereinrichtungen sind am Markt erhältlich und dem Fachmann bekannt.

Mit der zuvor beschriebenen Ausführungsform, d.h. der Regelung und Anpassung der Kompressorleistung, kann sichergestellt werden, dass die Trennkapazität der Membrantrennanlage den Bedürfnissen der Produktion des Rohgases und / oder notwendigen Mengen an Produktgasen (8) und (11) angepasst wird. Schwankungen in der Gaszusammensetzung der Ströme (8) und (11), die sich aus der unterschiedlich verarbeitenden Menge an Rohgas (17) und dessen Zusammensetzung ergeben, werden von den Steuereinrichtungen (18) und (19) ausgeglichen.

In einer weiteren bevorzugten Ausführungsform ist die erfindungsgemäße Vorrichtung derart ausgestaltet, dass sich ändernde Mengen an rückgeführtem Gas aus dem zweiten Permeatstrom (9b) und dem dritten Retentatstrom (10b), bevorzugt automatisch, mit einer Regelung der zugeführten Menge an Rohgas aus dem Rohgasstrom (17) ausgeglichen werden. Besonders bevorzugt erfolgt dies ohne dabei die Drehzahl des Kompressors zu ändern. Dies erlaubt die Verwendung einfacherer und kostengünstigerer, nicht regelbarer Kompressoren.

Als Messeinrichtungen (22) und (23) können in dieser Ausführungsform dabei Gassensoren, Volumen oder Massedurchflussmesser oder Druckmesser im zweiten Permeatstrom (9b) und/oder dem dritten Retentatstrom (10b) verwendet werden. Die Steuerung der zugeführten Menge an Rohgas erfolgt bevorzugt mittels einer Rohgasstromsteuereinrichtung (25) im Rohgasstrom. Die Rohgasstromsteuereinrichtung muss in der Lage sein, die fehlende Menge (= Differenz zwischen angesaugter Menge des Verdichters und Summe von rückgeführten Strömen (9b) und (10b)) zu ersetzen. Dies geschieht zum Beispiel durch Ausführung der Rohgasstromsteuereinrichtung als Druckmessung auf der Saugseite des Verdichters (4). Eine von dieser Druckmessung gesteuerte Dosiervorrichtung (z.B. ein analoges verstellbares Ventil oder ein Gebläse oder eine Verdichtungseinheit) kann nun den Druck konstant halten über unterschiedlich zugeführte Mengen an Rohgas (17). Steht das Rohgas unter einem Druck, der den Spezifikationen des Ansaugdruckes des Kompressors entspricht, so kann die zusätzlich zu den Rückströmen (9b) und (10b) benötigte Menge an Rohgas auch ohne zusätzliche Rohgassteuereinrichtung (17) direkt vom Kompressor angesaugt werden. Auch hierbei kann eine Datenverarbeitungseinrichtung zwischen die Messeinrichtungen (22) und (23) sowie der Rohgasstromsteuereinrichtung (25) zwischen geschaltet werden.

Grundsätzlich kann es sich bei den zuvor erwähnten Datenverarbeitungseinrichtungen um unterschiedliche Einrichtungen handeln, d.h. es können im erfindungsgemäßen Verfahren mehrere Datenverarbeitungseinrichtungen verwendet. Diese Datenverarbeitungseinrichtungen können optional miteinander vernetzt werden. Bevorzugt wird jedoch nur eine zentrale Datenverarbeitungseinrichtung verwendet mit der zentral alle Mess- und Steuerungsschritte überwacht und geregelt werden.

Die erfindungsgemäße Vorrichtung, siehe beispielhaft Abbildung 1, beinhaltet wie zuvor gesagt eine Verkettung von zumindest drei Membrantrennstufen. Jede Stufe besteht aus einem oder mehreren physikalischen Gasseparationsmodulen, die innerhalb einer Stufe parallel und/oder seriell verschaltet sind. Als Triebkraft für die Gasauftrennung in den Modulen wird eine Partialdruckdifferenz zwischen der Retentat- und der Permeatseite in den jeweiligen Membrantrennstufen erzeugt. Die Partialdruckdifferenz wird mittels eines Kompressors (4), welcher auf der Feedseite der Feedstromtrennstufe (1) angeordnet ist, und gegebenenfalls mittels zumindest einer, bevorzugt einer oder zwei Vakuumpumpe(n)(in Abbildung 1 nicht dargestellt) nach der Feedstromtrennstufe (1), bevorzugt auf der Permeatseite der Retentattrennstufe (2) im zweiten Permeatstrom (9a + 9b) und/oder auf der Permeatseite Permeattrennstufe (3) in dritten Permeatstrom (11), erzeugt. Ggf. kann es vorteilhaft sein in einer oder mehrerer der Membrantrennstufen die Partialdruckdifferenz durch einen permeatseitigen Spülgasstrom zu erzeugen bzw. zu verstärken.

In einer bevorzugten Ausgestaltungsform der vorliegenden Erfindung bringt ein Kompressor (4) das Rohgasgemisch bzw. das Gasgemisch aus dem Rohgasstrom (17) und dem zweiten Permeatstrom (9b) und dem dritten Retentatstrom (10b), auf den gewünschten Druck im Bereich von 5 bis 100 bar, vorzugsweise jedoch auf einen Druck von 9 bis 75 bar. Sollte der Rohgasstrom (17) schon den benötigten Druck aufweisen, so muss der Verdichter (4) nur mehr den zweiten Permeatstrom (9b) und den dritten Retentatstrom (10b) auf den gewünschten Druck im Bereich von 5 bis 100 bar, vorzugsweise jedoch auf einen Druck von 9 bis 75 bar bringen. Der erhaltene Feedstrom (5) wird in die Feedstromtrennstufe (1) eingeleitet. In der Feedstromtrennstufe (1) wird eine Vortrennung des Rohgasgemisches in leichter permeierende Komponenten (Permeatgas), die zu einem großen Teil ins Permeat der ersten Stufe gelangen und weniger schnell permeierende Komponenten (Retentatgas), die von der Membran vorwiegend zurückgehalten und sich im Retentat anreichern, erhalten. In einer bevorzugten Ausführungsform zeichnet sich das erfindungsgemäße Verfahren bzw. die erfindungsgemäße Vorrichtung dadurch aus, dass es/sie derart ausgestaltet ist, dass die Konzentration mindestens eines Permeatgases der Feedstromtrennstufe (1), nach Rückführung des zweiten Permeatstroms (9b) und des dritten Retentatstroms (10b), im Feedstrom (5)erhöht wird, bevorzugt um mindestens 2%, besonders bevorzugt um mindestens 3% und ganz besonders bevorzugt um 3 bis 40%, jeweils im Vergleich zur Konzentration im Rohgasstrom (17). Die Erhöhung kann von der Zusammensetzung des Rohgasstromes (17)abhängen und ist besonders ausgeprägt bei niedrigen Konzentrationen eines Permeatgases (10 bis 20%). Bevorzugt beträgt die Konzentrationserhöhung eines der Permeatgase zwischen 2 und 15% besonders bevorzugt zwischen 3 und 8 %, wenn der Gehalt des Permeatgases im Rohgasstrom (17) zwischen 30 und 70% beträgt. Es hat sich gezeigt, dass die Ausbeute des gesamten Prozesses an Retentatgas zunimmt und damit der Verlust an Retentatgas abnimmt, wenn die Konzentration des Permeatgases in der Feedstromtrennstufe (1) erhöht wird. Bei gleichem Stufentrennschnitt (=Verhältnis Permeatstrom zu Feedstrom der betrachteten Stufe) gelangt deutlich weniger Permeatgas ins Permeat der Feedstromtrennstufe (1), wenn die Konzentration mindestens einer in der Feedstromtrennstufe (1) leichter permeierenden Komponente A oder eines Permeatgases A im Feedstrom (5) erhöht wird. Analog wurde eine Erniedrigung festgestellt, wenn die Konzentration der Komponente A oder eines Permeatgases A im aufzureinigenden Feedstrom (5) sich verringert. So beträgt der Stufentrennschnitt für eine Konzentration von 50% einer Komponente A oder eines Permeatgases A im aufzureinigenden Feedstrom (5) zwischen 10 und 60%, vorzugsweise zwischen 15 und 55% und besonders bevorzugt zwischen 20 und 50 %. In einer besonders bevorzugten Ausführungsform der vorliegenden Erfindung ist das erfindungsgemäße Verfahren bzw. die erfindungsgemäße Vorrichtung daher derart ausgestaltet, dass der Gehalt an Permeatgas(en) der Feedstromtrennstufe (1) im Feedstrom (5) bei größer gleich 40 Vol. %, bevorzugt mehr als 50 Vol.% und ganz besonders bei mehr als 55 Vol. % bezogen auf das Volumen des Feedstroms (5), nach Rückführung des zweiten Permeatstroms (9b) und des dritten Retentatstroms (10b), liegt. Durch diese Konzentrationserhöhung der Permeatgase im Feedstrom (5) wird wie bereits erläutert die Effizienz der Feedstromtrennstufe (1) erhöht, was wiederum zur Folge hat, dass weniger Retentatgas B in den ersten Permeatstrom (6) gelangt. Dies erhöht wiederum die Effizienz der Permeattrennstufe (3) und sorgt dafür, dass auch hier weniger unerwünschtes Retentatgas in den dritten Permeatstrom (10a + b) gelangt. Insbesondere bei der Trennung von methanhaltigen Rohgasen führt dies zu dem Vorteil, dass die unerwünschten Emissionen des klimaschädlichen Methans deutlich reduziert werden konnten.

Allgemein kann man sagen, dass in der Feedstromtrennstufe (1) bevorzugt 20 bis 100%, besonders bevorzugt 40 bis 70 % der leichter permeierenden Komponente A, d.h. des Permeatgases A, vom Feedstrom (5) in das Permeat übergehen.

Das Retentat der Feedstromtrennstufe (1) wird, optional mit Druckminderung durch ein optional vorhandenes Druckminderventil (12) oder mit Druckerhöhung, mittels des ersten Retentatstroms (7) der Retentattrennstufe (2) zugeführt, in der die Feinreinigung erfolgt. Auf der Retentatseite der Retentattrennstufe (2), d.h. im zweiten Retentatstrom (8), befindet sich bevorzugt ein Druckminderventil (13) (in Abbildung 1 nicht gezeigt), mittels dessen der Hauptdruck im System (Betriebsdruck der Trennstufen (1) und (2) = Retentatdruck der Stufen (1) und (2)) aufrecht und konstant gehalten werden kann. Der Gehalt der schwerer permeierenden Komponenten B, d.h. eines Retentatgases B, wird in der Retentattrennstufe (2) weiter erhöht, sodass der Gehalt an Komponente B oder eines Retentatgases B im zweiten Retentatstrom (8) mehr als 90%, bevorzugt mehr als 95% und besonders bevorzugt mehr als 97% beträgt. In einer besonders bevorzugten Variante zeichnet sich dass erfindungsgemäße Verfahren bzw. die erfindungsgemäße Vorrichtung somit dadurch aus, dass mindestens 95%, bevorzugt mindestens 97%, besonders bevorzugt mindestens 99% und ganz besonders bevorzugt mindestens 99,5 %, der mit dem Rohgasstrom (17) in die Vorrichtung eingeführten Retentatkomponente der Feedstromtrennstufe (1) über den zweiten Retentatstrom (8) ausgeschleust werden.

Der Stufentrennschnitt der Retentattrennstufe (2) beträgt bei einer Konzentration der Komponente A oder eines Permeatgases A von 50% im ersten Retentatstrom (7) zwischen 10 und 60%, vorzugsweise zwischen 20 und 50 %.

Das Permeat der Retentattrennstufe (2) wird mittels des zweiten Permeatstroms (9b) besonders bevorzugt ohne dass zuvor Anteile des Permeatstroms (9a oder 9b) hinter der Feedstromtrennstufe (1) dem ersten Retentatstrom (7) zugeführt werden, ganz besonders bevorzugt vollständig,rückgeführt, dem Feedstrom (5) zugeführt und wiederaufbereitet. Dies kann - wie zuvor bei der Definition des Begriffes "Feedstrom" bereits erläutert - je nachdem ob ein Kompressor (4) oder gar ein mehrstufiger Kompressor (4) verwendet wird auf unterschiedliche Art und Weise erfolgen. Bei einem einstufigen Kompressor (4) wird der zweite Permeatstrom (9b) bevorzugt der Saugseite des Kompressors (4) zugeführt.

Das mit der Komponente A oder einem Permeatgas A stark angereicherte Permeat der Feedstromtrennstufe (1) wird mittels des ersten Permeatstroms (6) der Permeattrennstufe (3) zugeführt. Es ist notwendig mittels der Retentatsteuereinrichtung (19) im Retentatstrom der Permeattrennstufe (3), d.h. dem dritten Retentatstrom (10a + b)), zu verhindern, dass der Druck des Retentats der Permeattrennstufe (3) auf Umgebungsdruck abfällt. Auf diese Weise kann die treibende Kraft für die Permeattrennstufe (3) erhalten bleiben. Die Permeattrennstufe (3) produziert ein Permeat mit einem Gehalt an besser permeabler Komponente A oder eines Permeatgases A größer als 95%, vorzugsweise größer als 97%
und besonders vorzugsweise größer als 99%, welches über den dritten Permeatstrom (11) aus der Vorrichtung ausgeschleust wird. In einer besonders bevorzugten Ausführungsform ist die erfindungsgemäße Vorrichtung derart ausgestaltet, dass maximal 5%, bevorzugt maximal 3%, besonders bevorzugt maximal 1% und ganz besonders bevorzugt maximal 0,5 %, der mit dem Rohgasstrom (17) in die Vorrichtung eingeführten langsamer permeierenden Komponente B der Feedstromtrennstufe (1) über den dritten Permeatstrom (11) ausgeschleust werden.

Der Stufentrennschnitt der Permeattrennstufe (3) beträgt bevorzugt zwischen 50 und 95%, und besonders bevorzugt zwischen 70 und 93%.

Der dritte Retentatstrom (10b) wird rückgeführt, dem Feedstrom (5) zugeführt und wiederaufbereitet. Dies kann auf unterschiedliche Weise erfolgen und kann z. B. davon abhängen, ob ein Kompressor (4) oder gar ein mehrstufiger Kompressor (4) verwendet wird. Bei einem einstufigen Kompressor (4) wird der dritte Retentatstrom (10b) bevorzugt der Saugseite des Kompressors (4) zugeführt, wenn der Ansaugdruck des Kompressors kleiner ist als der Retentatdruck der Trennstufe (3). Wird ein mehrstufiger Kompressor verwendet, so ist es bevorzugt, dass der dritte Retentatstrom (10b) zwischen zwei Verdichtungsstufen in den Kompressor eingeführt wird, wenn der Stufen druck des Kompressor bei der jeweiligen Stufe kleiner ist als der Retentatdruck der Trennstufe (3).

Das erfindungsgemäße Verfahren bzw. die erfindungsgemäße Vorrichtung zeichnet sich in einer weiteren bevorzugten Ausführungsform besonders dadurch aus, dass es/sie derart ausgestaltet ist, dass das im zweiten Permeatstrom (9b) und im dritten Retentatstrom (10b) zurückgeführte Gasvolumen in Summe weniger als 60 Vol. %, bevorzugt 10 bis 50 Vol. %, ganz besonders bevorzugt 20 bis 40 Vol. % des Volumens des Rohgasstroms (17) beträgt. Die Steuerung der Menge der rückzuführenden Retentatgasströme hängt von den geforderten Reinheiten in den Produktgasströmen (8) und (11) ab. Je geringer die angeforderten Reinheiten sind, umso geringer werden die Rückströme (9b) und (10b). Die Rückströme werden ganz besonders durch die Art und die Selektivität der eingesetzten Membranmodule in den Membrantrennstufen (1) bis (3) beeinflusst. Membranmodule mit erhöhter Selektivität bewirken dabei eine deutliche Senkung der Rückströme (9b) und (10b). Auch der Hauptdruck im System (= Druck in den Trennstufen (1) und (2)) beeinflussen die Menge an rückgeführten Gasen. Je höher der Druck im System ist, umso geringer werden die rückgeführten Mengen. Ein weiterer Einflussfaktor sind die Verhältnisse an Membranfläche in den einzelnen Stufen. Größere Flächen in der Trennstufe (3) verringern
zum Beispiel die Rückströmung, größere Flächen in der Trennstufe (2) erhöhen hingegen die Rückströmungen. Somit zeichnet sich das erfindungsgemäße Verfahren bzw. die Vorrichtung dadurch aus, dass trotz sehr geringer Rückströme die oben näher erläuterte Erhöhung der Konzentration der Permeatkomponente im Feedstrom (5) sichergestellt wird. Dies erhöht deutlich die Effizienz des gesamten Verfahrens.

Wie bereits erläutert ist es besonders vorteilhaft wenn ein mehrstufiger Kompressor (4) eingesetzt wird. In diesem Fall kann nämlich auf eine komplette Entspannung des Retentats der Permeattrennstufe (3) verzichtet werden kann, da das Retentat der Permeattrennstufe (3) zwischen zwei Verdichterstufen des Kompressors (4) eingespeist werden kann. Da die Retentattrennstufe (2) bei Entspannung auf Feeddruck im Regelfall im selektivitätslimitierten Bereich betrieben werden würde, kann es sinnvoll sein den zweiten Permeatstrom (9a) lediglich auf ein höheres Druckniveau einer mehrstufigen Druckerhöhungseinheit, d.h. eines mehrstufigen Kompressors (4) zu entspannen da sich somit die Betriebskosten der Kompressionseinheit reduzieren ohne das Trennergebnis deutlich zu verschlechtern. In einer besonders bevorzugten Ausführungsform der vorliegenden Erfindung wird daher ein mehrstufiger Kompressor (4) verwendet und die Gasströme (9b) und (10b) diesem Kompressor jeweils zwischen zwei Kompressionsstufen zugeführt.

In einer bevorzugten Ausführungsform wird der Druckabfall über die Feedstromtrennstufe (1) auf 1 und 30 bar, vorzugsweise auf 2 und 20 bar und besonders vorzugsweise zwischen 3 und 10 bar beschränkt.-Gleichzeitig oder alternativ wird bevorzugt sichergestellt, dass der Druckabfall über die Feedstromtrennstufe (1) und die Retentattrennstufe (2), auf 1 und 100 bar, vorzugsweise zwischen 5 und 80 bar und besonders vorzugsweise zwischen 10 und 70 bar beschränkt wird.

Die erfindungsgemäße Vorrichtung bzw. das erfindungsgemäße Verfahren kann im Prinzip mit allen Membranen realisiert werden, die in der Lage sind binäre Gasgemische oder Multigasgemische zu trennen. Als Membranmaterialien kommen bevorzugt aber nicht ausschließlich Kunststoffe zum Einsatz. Als Kunststoffe in der trennaktiven Schicht kommen besonders bevorzugt Polyimide, Polyamide, Polysulfone, Celluloseacetate und Derivate, Polyphenylenoxide, Polysiloxane, Polymere mit intrinsischer Mikroporosität, Mixed Matrix Membranen, Facilitated Transport Membranen, Polyethylenoxide, Polypropylenoxide, Kohlenstoffmembranen oder Zeolithe oder Mischungen daraus in Frage.

Besonders bevorzugte Membranen weisen als Materialien für die trennaktive Schicht bzw. als Material für die komplette Membran ein Polyimid der allgemeinen Formel 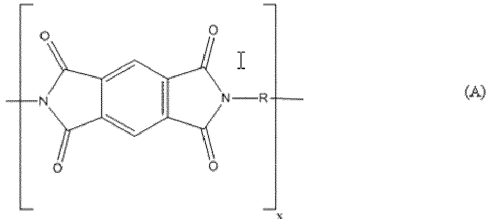 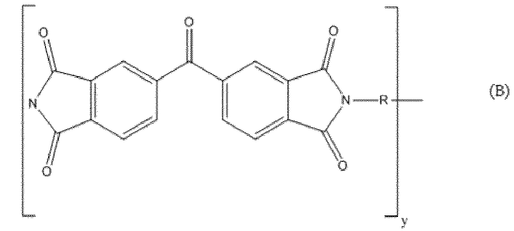 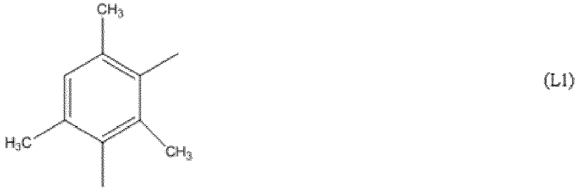 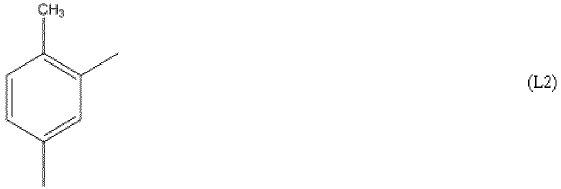 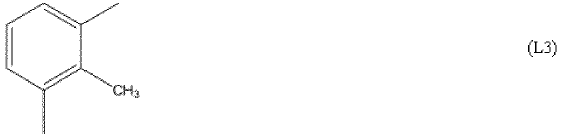 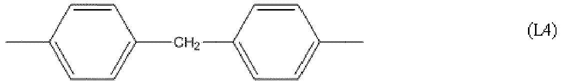 mit 0 ≤ x ≤ 0,5 und 1 ≥ y ≥ 0,5 und R entspricht einem oder mehreren, gleichen oder unterschiedlichen Resten R, ausgewählt aus der Gruppe bestehend aus den Resten L1, L2, L3 und L4, auf.

Besonders bevorzugt handelt es sich um ein Polymer mit x = 0, Y = 1 und R bestehend zu 64 mol % aus L2, zu 16 mol % aus L3 und zu 20 mol % aus L4. Dieses Polymer ist unter dem Namen P84 oder P84 Typ 70 (CAS- Nummern 9046-51-9) von der Firma Evonik Fibres GmbH erhältlich. Speziell bevorzugt wird ein Polymer mit der Zusammensetzung x = 0,4, y = 0,6 und R bestehend zu 80 mol % aus L2, zu 20 mol % aus L3, verwendet. Dieses Polymer ist unter dem Namen P84HT oder P84 HT 325 (CAS- Nummern 134119-41-8) von der Firma Evonik Fibres GmbH erhältlich. Ebenfalls bevorzugt können Mischungen besagter Polyimide verwendet werden.

Aus den bevorzugten Polyimiden hergestellte Membranen sind von der Firma Evonik Fibres GmbH unter dem Namen Sepuran erhältlich. Ein Verfahren zur Herstellung dieser bevorzugten Membranen wird in der WO 2011/009919 A1 offenbart. Sämtliche in dieser Offenlegungsschrift offenbarten Membranen können im erfindungsgemäßen Verfahren bevorzugt eingesetzt werden. Zur Vermeidung reiner Wiederholungen wird auf den Inhalt dieser Patentanmeldung hiermit vollumfänglich Bezug genommen. Es wurde gefunden, dass mit diesen Membranen die besten Trennergebnisse erzielt werden können.

Die Membranen werden bevorzugt in Form von Hohlfasermembranen und / oder Flachmembranen verwendet. Die Membranen werden zu Modulen verbaut, die dann in der Trennaufgabe zum Einsatz kommen. Als Module können alle in der Technik bekannten Gasseparationsmodule wie zum Beispiel aber nicht ausschließlich Hohlfasergasseparationsmodule, Spiralwickelgasseparationsmodule, Kissengasseparationsmodule oder Rohrbündelgasseparationsmodule zum Einsatz kommen.

Die Gasseparationsmembranmodule haben erfindungsgemäß eine Gemischtgasselektivität der Komponenten A (CO₂) und B (CH₄) (= Verhältnis des Stoffstromes A zum Stoffstrom B über die Membran) von mindestens 30, bevorzugt mindestens 35, besonders bevorzugt mindestens 40, ganz besonders bevorzugt von mindestens 45 und speziell bevorzugt von mindestens 45 bis 80. Höher selektive Membranen haben den Vorteil, dass die Trennung effektiver wird und weniger Permeat aus Retentattrennstufe (2) bzw. weniger Retentat aus der Permeattrennstufe (3) rückgeführt werden muss. Damit muss insbesondere bei Verwendung eines einstufigen Kompressors (4) weniger Gas doppelt komprimiert werden, was wirtschaftliche Vorteile beim Betrieb der Anlage mit sich bringt. Bei sehr selektiven Membranmodulen mit einer Selektivität von 45 müssen nur ca. 35% des als Rohgas in die Feedstromtrennstufe (1) eingebrachten Gases doppelt komprimiert werden, mit einem Membranmodul mit einer Selektivität von nur 10 kann es sein, dass die Doppelkomprimierung bis zu 300% beträgt. Die Angaben 35% bzw. 300% beziehen sich auf Versuche, bei denen ein Gasgemisches mit äquimolaren Mengen an Komponente A und B (= Feed) aufgegeben wurden, wobei 98,5% Komponente B im Retentatgas der Stufe(2) und 99% an Komponente B im Permeatstrom der Stufe (3) enthalten waren.

Es ist augenscheinlich, dass der erfindungsgemäße Prozess mit selektiveren Membranen wesentlich wirtschaftlicher geführt werden kann und die notwendige Größe des Kompressors und die benötigten Energie reduziert werden können.

Das erfindungsgemäße Verfahren / die erfindungsgemäße Vorrichtung hat insbesondere die Vorteile, dass es ein reines Membranverfahren ist und ohne zusätzliche Aufreinigung der Permeat- und/oder Retentatströme (11) bzw. (8) für viele Anwendungen auskommt. Man braucht zum Bespiel bei der Aufreinigung von Biogas oder Erdgas (= Abtrennung von Kohlendioxid aus Methan) keine Druckwechseladsorption oder Aminwäsche mehr zur Feinreinigung des Retentates, sodass dieses ins Erdgasnetz eingespeist werden kann. Weiterhin kann mit dem erfindungsgemäßen Verfahren / der erfindungsgemäßen Vorrichtung gleichzeitig ein reiner Retentatstrom (8) und ein reiner Permeatstrom (11) bei der Bio- und Erdgasaufreinigung hergestellt werden. Es kann daher ohne große Verluste an Methan und ohne große Beeinträchtigung der Umwelt in die Atmosphäre entlassen werden, ohne dass
das Gas noch weiter behandelt werden muss durch eine katalytische oder thermische Nachverbrennung oder eine Nutzung in einem Blockheizkraftwerk. Es entfällt daher die Investition in weitere Anlagenteile, was zu einem wirtschaftlicheren Aufreinigungsprozess für Bio- und Erdgas führt.

Die erfindungsgemäße Vorrichtung ist in großen Zügen bereits in der WO 2012/000727 beschrieben. Der Gegenstand der WO 2012/000727 wird daher vollumfänglich in die Beschreibung der vorliegenden Erfindung mit einbezogen.

In der WO 2012/000727 wird keine Steuerung offenbart, die es erlauben würde Schwankungen der Zusammensetzung oder des Druckes oder des Flusses des Rohgasstromes auszugleichen. Die WO 2012/000727 offenbart lediglich die Variation der Kompressorleistung und allgemeine Druckbereiche welche eingehalten werden sollten um eine gute Ausbeuten und Reinheiten der Produktgase zu erhalten. Mit der vorliegenden Erfindung wird erstmal ein Steuerungs- und Regelungskonzept einer Membranverschaltung gemäß WO 2012/000727 offenbart, welche es erlaubt diese Aufreinigungsanlage auch an Biogasanlagen mit variablem Feedstrom direkt anzuschließen. Es kann daher auf spezielle Voreinrichtungen verzichtet werden, welche einen annähernd konstanten Rohgasstrom bereitstellen. Die vorliegende Erfindung stellt daher eine bedeutende Weiterentwicklung der Anlage und des Verfahrens der WO 2012/000727 dar. Dies gilt insbesondere, da durch die Steuereinrichtungen (18) und (19) und deren erfindungsgemäßer Regelung, eine konstante oder auch auf Grund wechselnder Anforderungen wechselnde Gasqualität der Ströme (8) und (11) garantiert werden kann, auch wenn sich die zu verarbeitende Menge an Rohgas (17) und/oder die herzustellende Menge an Produktgasen (8) und/oder (11) und/oder die Rohgaszusammensetzung ändert. Hierbei ist es vorteilhaft, dass sich zur Einhaltung der geforderten Gasqualitäten in den Strömen (8) und (11) der Hauptdruck im System (= Betriebsdruck in den Trennstufen (1) und (2)) und die Membraneflächen in den Trennstufen (1) bis (3) nicht geändert werden muss.

Ein weiterer Vorteil ist darin zu sehen, dass das erfindungsgemäße Verfahren / die erfindungsgemäße Vorrichtung
mit deutlich geringerem apparativem und energetischen Aufwand auskommt wie die bekannten Verfahren des Standes der Technik.

Die erfindungsgemäße Vorrichtung bzw. das erfindungsgemäße Verfahren kann insbesondere zur Auftrennung von Gasgemischen mit mindestens zwei Gasen eingesetzt werden, wobei ganz besonders bevorzugt als Gasgemisch eine Mischung von vorwiegend aber nicht ausschließlich Kohlendioxid und Methan oder vorwiegend aber nicht ausschließlich Wasserstoff und Methan oder vorwiegend aber nicht ausschließlich Kohlenmonooxid und Wasserstoff oder Rohbiogas oder Roherdgas aufgetrennt wird. Die nachfolgenden Beispiele sollen die vorliegende Erfindung näher erläutern und beschreiben, schränken diese jedoch in keiner Weise ein.

### Allgemeiner Versuchsaufbau

Es wurden Versuche an einer Membrantrennanlage vorgenommen, die eine dreistufige Verschaltung in Anlehnung an Abbildung 1 verwendet.
- Rohgaszusammensetzung 54% Methan, 46% CO₂ (= Biogas aus einer Biogasanlage)
- Verwendung von 3 Stück 2" Versuchsmodulen von Sepuran Green (je 1 Modul pro Stufe)
- Hauptdruck im System (= Druck Retentat Stufe (2)) betrug 17 bara
- Luftdruck 950 mbara
- Permeatdruck der Permeattrennstufe (3) lag bei 1000 mbara

### Beispiel 1:

Ziel dieses Versuches war es eine Kalibiergerade zu finden, die es erlaubt, durch Änderung des Permeatdruckes der Retentattrennstufe (2) die Produktgasqualität im Retentatstrom (8) und durch Änderung des Retentatdruckes der Permeattrennstufe (3) die Offgasqualität im Permeatstrom (11) konstant zu halten, wenn die Feedmenge im Feedstrom (5) bzw. die Kompressordrehzahl geändert wird.

Zu diesem Zwecke wurde bei einer laufenden 3 stufigen Verschaltung gemäß dem allgemeinen Versuchsaufbau die Kompressorleistung in Stufen erhöht. Es wurde nun durch Änderung der Drücke des Permeates der Retentattrennstufe (2) und des Retentates der Permeattrennstufe (3) versucht die Offgas- (11) und die Produktgaskonzentration (8) in einem engen Rahmen konstant zu halten. Durch die Erhöhung der Kompressorleistung von anfangs 60% auf zum Ende 75% erhöht sich der Feedvolumenstrom (5) von 3,83 m³/h auf 5,23 m³/h oder um 36%. In diesem Intervall verringert sich der Permeatdruck der Retentattrennstufe (2) von 951 mbara auf 241 mbara und der Retentatdruck der Permeattrennstufe (3) erhöht sich von 3,6 bara auf 4,43 bara. Die Produktgaskonzentration (8) bewegt sich bei allen Kompressorleistungen zwischen 95,23 und 95,75 % Methan, die Offgaskonzentration an Methan zwischen 0,5 und 0,62%, Beide Werte sind im Rahmen der Messungenauigkeit in einem engen Bereich geregelt. Detaillierte Daten zu diesem Versuch finden sich in der nachfolgenden Tabelle 1:

**Tabelle 1:**

| Kompressorleistung [%] | Fluss Feedstrom (5) [m³/h] | Druck Perm.-strom (9a) [mbara] | Druck Ret.-strom (10a) [bara] | c(CH₄) Retstrom (8) [%] | c(CH4) Perm.-strom (11) [%] | Fluss Ret.-strom (8) [m³/h] | Fluss Perm .-strom (9a) [m³/h] | Fluss Perm .-strom (6) [m³/h] | Fluss Perm .-strom (11) [m³/h] | Fluss Ret.-strom (7) berechnet [m³/h] |
|---|---|---|---|---|---|---|---|---|---|---|
| 60 | 3,83 | 951 | 3,6 | 95,75 | 0,5 | 1,665 | 0,622 | 1,641 | 1,28 | 2,287 |
| 62,5 | 4,1 | 760 | 3,8 | 95,68 | 0,62 | 1,807 | 0,756 | 1,669 | 1,372 | 2,563 |
| 65 | 4,3 | 660 | 3,9 | 95,54 | 0,58 | 1,907 | 0,838 | 1,715 | 1,427 | 2,745 |
| 67,5 | 4,53 | 560 | 4,03 | 95,23 | 0,62 | 2 | 0,94 | 1,76 | 1,5 | 2,94 |
| 70 | 4,77 | 460 | 4,16 | 95,52 | 0,55 | 2,086 | 1,044 | 1,828 | 1,57 | 3,13 |
| 72,5 | 5,01 | 320 | 4,29 | 95,43 | 0,55 | 2,175 | 1,16 | 1,894 | 1,646 | 3,335 |
| 75 | 5,23 | 241 | 4,43 | 95,34 | 0,62 | 2,267 | 1,28 | 1,925 | 1,697 | 3,547 |

Weiters wurden die Volumenströme des zweiten Retentatstroms (8), des ersten Permeatstroms (6), des dritten Permeatstroms (11) (= Offgas) und des zweiten Permeatstroms (9a) gemessen. Aus der Summe der Volumenstromwerte des zweiten Retentatstroms (8) und des zweiten Permeatstroms (9a) lassen sich die Volumenströme des ersten Retentatstroms (7) ermitteln.

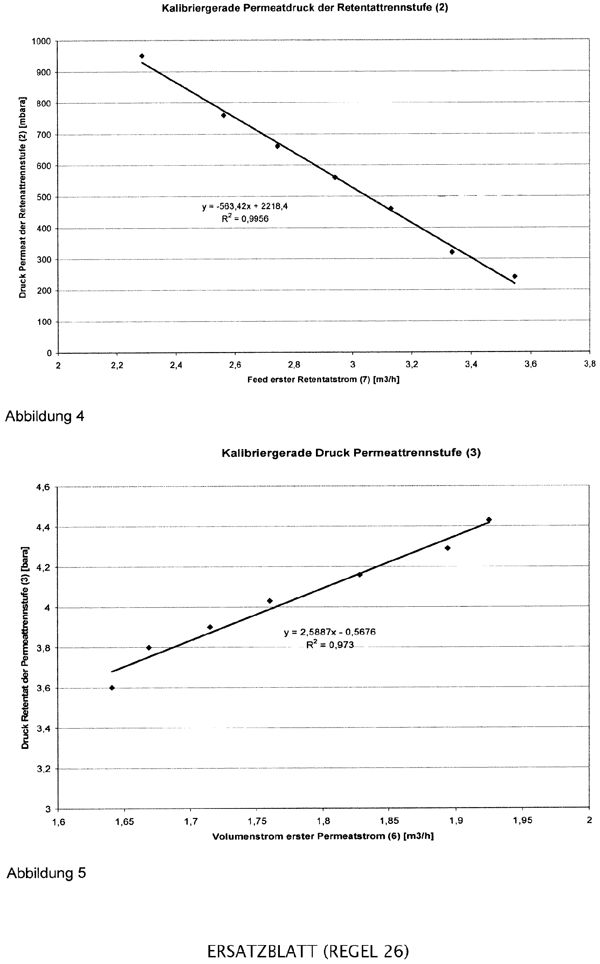

Durch Auftragung des Permeatdruckes der Retentattrennstufe (2) in Abhängigkeit vom Volumenstrom des ersten Retentatstroms (7) lässt sich nun eine Kalibrierkurve ermitteln, um die Produktgaskonzentration konstant zu halten, wenn sich die Feedmenge der Retentattrennstufe (2) ändert, z. B. durch eine Änderung der Kompressordrehzahl oder durch eine Änderung in der Zusammensetzung des Rohgases (siehe Abbildung 4).

Wie man Abbildung 4 entnehmen kann erhält man Messpunkte mit einer linearen Zusammensetzung guter Korrelation. Diese Beziehung kann nun genutzt werden, um in eine Steuerung der erfindungsgemäßen Anlage eingesetzt zu werden. Diese Steuerung ermittelt nach Ermittlung eines Flusswertes des ersten Retentatstroms (7), gemessen mittels eines Volumenstrommessers (20a), durch Errechnung des Permeatdrucks nach der Geradengleichung in Abbildung 4 den notwendigen Permeatdruck in der Retentattrennstufe (2), um die Produktgaskonzentration konstant zu halten. Dieser Druck wird dann mit einer Steuereinrichtung (18) im zweiten Permeatstrom entsprechend eingestellt.

Analog erhält man durch Auftragung des Retentatdruckes der Permeattrennstufe (3) in Abhängigkeit der Menge des ersten Permeatstroms (6) auch eine Kalibrierkurve, um die Offgaskonzentration im Permeatstrom (11) konstant zu halten, wenn sich die Feedmenge in die Permeattrennstufe (3), d.h. der erste Permeatstrom (6), ändert, z.B. durch eine Änderung der Kompressordrehzahl oder durch eine Änderung in der Zusammensetzung des Rohgases (siehe Abbildung 5).

Man erhält auch in Abbildung 5 Messpunkte mit einer linearen Zusammensetzung guter Korrelation. Diese Beziehung kann nun analog zum oben beschriebenen Vorgehen für die Rententattrennstufe (2) genutzt werden, um in eine Steuerung der erfindungsgemäßen Anlage eingesetzt zu werden. Dabei wird zunächst der Flusswert des ersten Permeatstroms (6), gemessen mittels eines Volumenstrommessers (21a), ermittelt und aus der Geradengleichung in Abbildung 5 der notwendige Retentatdruck in der Permeattrennstufe (3) ermittelt und mittels der Steuereinrichtung (19) im dritten Retentatstrom (10) eingestellt, um die Offgaskonzentration im Permeatstrom (11) konstant zu halten.

### Beispiel 2:

Es soll überprüft werden, ob durch eine Änderung des Retentatdrucks der Permeattrennstufe (3), mittels der Steuereinrichtung (19) im dritten Retentatstrom (10), eine Änderung der Methankonzentration im Offgas der Anlage (dritter Permeatstrom (11)) erreicht werden und eine Kalibrierkurve erhalten werden kann. Sollte bei einer Messung der Offgaskonzentration eine Änderung auftreten, so könnte dann durch zu Hilfenahme dieser Kalibrierbeziehung der Methangehalt im Offgas angepasst werden.

Zu diesem Zweck wurde bei konstanter Kompressordrehzahl der Retentatdruck der Permeattrennstufe (3) durch eine Steuereinrichtung (19) in dritten Retentatstrom (10) geändert und die sich dabei ändernde Methankonzentration im dritten Permeatstrom (11) (Offgas) gemessen. Auch die Volumenströme der Anlage wurden mit aufgezeichnet. Die Werte sind in Tabelle 2 dargestellt.

**Tabelle 2:**

| Komprleistung [%] | Fluss Feed - strom (5) [m³/h] | Druck Ret.-strom (10a) (bara) | Druck Perm.-strom (9a) (mbara) | c(CH4) Perm.-strom (11) [%] | Fluss Ret.-strom (8) [m³/h] | Fluss Perm. -strom (9a) [m³/h] | Fluss Perm. -strom (6) [m³/h] | Fluss Perm. -strom (11) [m³/h] | Fluss Ret.strom (7) berechnet [m³/h] | Doppelkomprimierung |
|---|---|---|---|---|---|---|---|---|---|---|
| 60 | 3,3 | 3,5 | 950 | 0,99 | 1,494 | 0,47 | 1,37 | 1,166 | 1,964 | 24,1% |
| 60 | 3,3 | 3,4 | 950 | 0,94 | 1,482 | 0,456 | 1,399 | 1,147 | 1,938 | 25,5% |
| 60 | 3,3 | 3,3 | 950 | 0,88 | 1,462 | 0,44 | 1,434 | 1,122 | 1,902 | 27,7% |
| 60 | 3,3 | 3,2 | 950 | 0,82 | 1,44 | 0,427 | 1,464 | 1,09 | 1,867 | 30,4% |
| 60 | 3,3 | 3,1 | 950 | 0,76 | 1,406 | 0,409 | 1,509 | 1,062 | 1,815 | 33,7% |
| 60 | 3,3 | 3 | 950 | 0,69 | 1,375 | 0,394 | 1,555 | 1,027 | 1,769 | 37,4% |
| 60 | 3,3 | 2,9 | 950 | 0,63 | 1,347 | 0,38 | 1,596 | 0,986 | 1,727 | 41,4% |
| 60 | 3,3 | 2,8 | 950 | 0,56 | 1,283 | 0,36 | 1,663 | 0,955 | 1,643 | 47,5% |
| 60 | 3,3 | 2,7 | 950 | 0,5 | 1,247 | 0,345 | 1,713 | 0,911 | 1,592 | 52,9% |
| 60 | 3,3 | 2,6 | 950 | 0,44 | 1,177 | 0,33 | 1,789 | 0,868 | 1,507 | 61,4% |

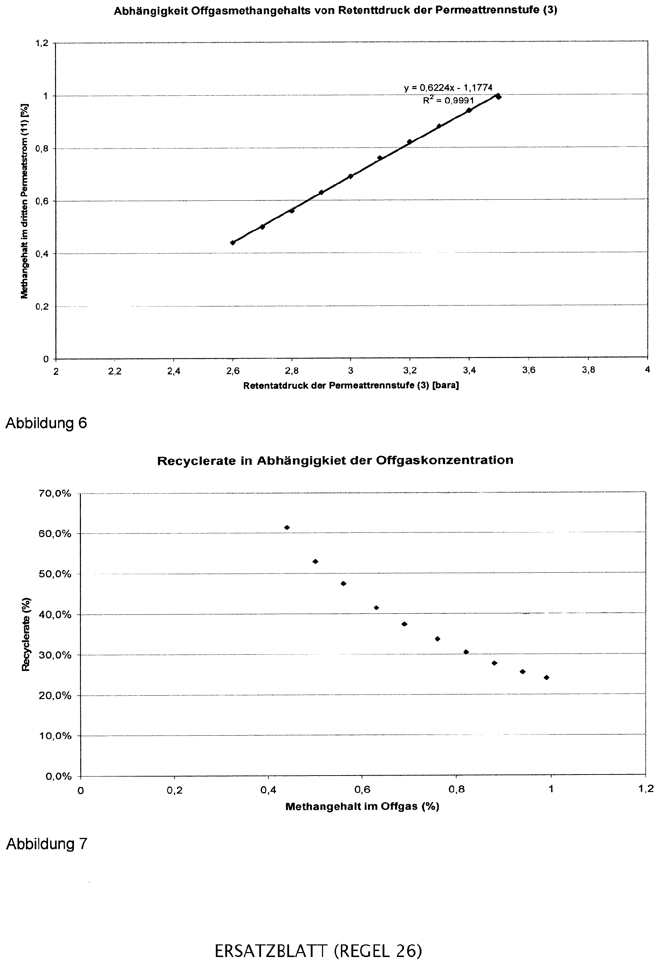

Wie Tabelle 2 zeigt erhöht sich die Methankonzentration im Offgasstrom (11) durch Erhöhung des Retentatdruckes in der Permeattrennstufe (3). Grafisch ist dies in Abbildung 6 zu sehen. Die Abhängigkeit ist hier linear mit sehr guter Korrelation. Diese Kurve kann als Kalibrierkurve für eine Steuerung verwendet werden. Durch Einsetzen der gewünschten Methankonzentration in die Gleichung in Abbildung 6 kann der hierzu erforderliche Retentatdruck ermittelt werden.

Interessant anzumerken ist die stark steigende Doppelkomprimierungsrate bei sinkendem Retentatdruck der Permeattrennstufe (3) und damit sinkender Methankonzentration im Offgas grafisch dargestellt in Abbildung 7.

### Beispiel 3:

Durch eine Änderung des Permeatdruckes der Retentattrennstufe (2) mittels einer Steuereinrichtung (18) im zweiten Permeatstrom (9a) kann eine Änderung der Methankonzentration im Produktgas der Anlage (= zweiter Retentatstrom (8)) erreicht werden. Sollte bei einer Messung der Produktgaskonzentration eine Änderung auftreten, so kann durch zu Hilfenahme dieser Kalibrierbeziehung der Methangehalt im Produktgas angepasst werden.

Zu diesem Zweck wurde bei konstanter Kompressordrehzahl der Permeatdruck der Retentattrennstufe (2) geändert und die sich dabei ändernde Methankonzentration im Produktgas gemessen. Die Werte sind in Tabelle 3 dargestellt.

**Tabelle 3:**

| Permeatdruck Stufe 2 [bara] | c(CH₄) im retenatstrom (8) [%] |
|---|---|
| 1,005 | 96,44 |
| 0,95 | 96,77 |
| 0,9 | 97,03 |
| 0,85 | 97,23 |
| 0,8 | 97,48 |
| 0,75 | 97,68 |
| 0,7 | 97,93 |
| 0,65 | 98,14 |
| 0,6 | 98,34 |
| 0,55 | 98,55 |
| 0,5 | 98,80 |
| 0,445 | 99,07 |
| 0,4 | 99,28 |
| 0,35 | 99,47 |
| 0,3 | 99,59 |
| 0,284 | 99,66 |

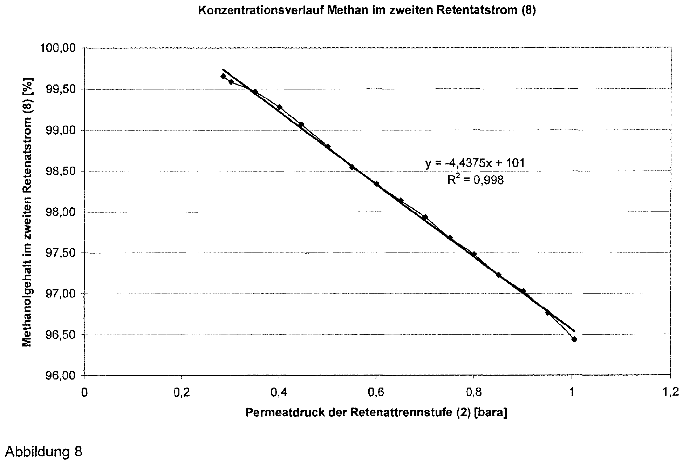

Wie man sieht erhöht sich die Methankonzentration im Produktgas (8) durch Erniedrigung des Permeatdruckes in der Retentattrennstufe (2). Grafisch ist dies in Abbildung 8 zu sehen. Die Abhängigkeit ist hier linear mit sehr guter Korrelation. Diese Kurve kann als Kalibrierkurve für eine Steuerung verwendet werden. Durch Einsetzen der gewünschten

Methankonzentration in die Gleichung in Diagramm 5 kann der hierzu erforderliche Permeatdruck ermittelt werden.

### Beschreibung der Abbildungen

- Abb.1:: Beispielhafte erfindungsgemäße Verschaltung mit den Messeinrichtungen (20a) und (20b), (21a) und (21b), (22) und (23) sowie den Steuereinrichtungen (18) und (19). Die Steuereinrichtung im Rohgasstrom (17) sowie die Kontroll- und Datenerarbeitungseinrichtungen sind nicht gezeigt. Aus dem Gesamtzusammenhang der Beschreibung geht deren Anordnung und Anwendung jedoch klar hervor. In Abbildung 1 wird eine erfindungsgemäße Anordnung mit der Rückführung der Ströme (9b) und (10b) auf die Saugseite des Kompressors gezeigt. Alternative in der obigen Beschreibung erläuterte Anordnungen wie z. B. die Rückführung einer oder mehrerer der Ströme (9b) oder (10b) in eine erhöhte Kompressionsstufe des Kompressors (4) oder ohne die Messeinrichtungen (22) und (23) oder mit nur einem Teil der Messeinrichtungen (20a) und (20b) und (21a) und (21b), sind für einen Fachmann leicht als Abwandlung von Abbildung 1 ableitbar und werden daher nicht separat gezeigt. Abbildung 1 dient lediglich der Erläuterung der vorliegenden Erfindung und schränkt deren Schutzumfang in keiner Weise ein.
- Abb. 2:: Gezeigt wird der notwendige Permeatdruck der Retentattrennstufe (2) in Abhängigkeit des Volumenstromes des ersten Retentatstroms (7) zur Erreichung einer Retentatqualität von 98,3 % der Komponente B im zweiten Retentatstrom (8) und von 0,7 % der Komponente B im dritten Permeatstrom (11). Das gewählte Flächenverhältnis der Membranen in den Membrantrennstufen war wie folgt: Stufe 1: Stufe 2: Stufe 3= 2:2:3. Es sind drei Kurven dargestellt für unterschiedliche Konzentrationen der Komponente B (in diesem Fall CH₄) von 45, 55 und 65% im Rohgasstrom (17)
- Abb. 3:: Gezeigt wird der notwendiger Retentatdruck der Permeattrennstufe (3) in Abhängigkeit des Volumenstromes des ersten Permeatstroms (6) zur Erreichung einer Retentatqualität von 98,3 % der Komponente B im zweiten
Retentatstrom (8) und von 0,7 % der Komponente B im dritten Permeatstrom (11). Das gewählte Flächenverhältnis dem Membranen in den Membrantrennstufen war wie folgt: Stufe 1: Stufe 2: Stufe 3= 2:2:3. Es sind drei Kurven dargestellt für unterschiedliche Konzentrationen der Komponente B (in diesem Fall CH₄) von 45, 55 und 65% im Rohgasstrom (17), die ineinander übergehen.
- Abb. 4:: Abhängigkeit des Permeatdruckes der Retentattrennstufe (2) von der Feedgasmenge der Retentattrennstufe (2) zur Konstanthaltung der Produktgasqualität
- Abb. 5:: Abhängigkeit des Retentatdruckes der Permeattrennstufe (3) von der Feedgasmenge der Permeattrennstufe (3) zur Konstanthaltung der Offgasqualität
- Abb. 6:: Abhängigkeit der Methankonzentration im Offgas (11) vom Retentatdruck der Permeattrennstufe (3)
- Abb. 7:: Abhängigkeit der Recyclingrate vom Methangehalt im Permeat (11) der Permeattrennstufe (3)
- Abb. 8:: Abhängigkeit der Methankonzentration im Produktgas (8) vom Permeatdruck der Retentattrennstufe (2)

### Bezugszeichenliste:

- 1:: Feedstromtrennstufe
- 2:: Retentattrennstufe
- 3:: Permeattrennstufe
- 4:: einstufiger oder mehrstufiger Kompressor
- 5:: Feedstrom
- 6:: erster Permeatstrom
- 7:: erster Retentatstrom
- 8:: zweiter Retentatstrom
- 9:: zweiter Permeatstrom bestehend aus den Teilströmen 9a, zwischen der Steuereinrichtung 18 und der Retentattrennstufe 2, und 9b stromabwärts der Steuereinrichtung 18
- 10:: dritter Retentatstrom bestehend aus den Teilströmen 10a, zwischen der Steuereinrichtung 19 und der Permeattrennstufe 3, und 10b stromabwärts der Steuereinrichtung 19
- 11:: dritter Permeatstrom
- 12:: Optionales Druckminderventil im ersten Retentatstrom 7 (in den Abbildungen nicht wiedergegeben)
- 13:: Optionales Druckminderventil im zweiten Retentatstrom 8 (in den Abbildungen nicht wiedergegeben)
- 14:: Optionales Druckminderventil im dritten Retentatstrom 10 (in den Abbildungen nicht wiedergegeben)
- 15:: Vakuumpumpe (in den Abbildungen nicht wiedergegeben)
- 16:: Mischkammer (in den Abbildungen nicht wiedergegeben)
- 17:: Rohgasstrom
- 18:: Permeatsteuereinrichtung im 2. Permeatstrom (in der Beschreibung auch einfach als Steuereinrichtung 18 bezeichnet)
- 19:: Retentatsteuereinrichtung im 3. Retentatstrom (in der Beschreibung auch einfach als Steuereinrichtung 19 bezeichnet)
- 20a:: 1. Retentatmesseinrichtung zur Analyse des 1. Retentatstroms (in der Beschreibung auch einfach als Messeinrichtung 20a bezeichnet)
- 20b:: 2. Retentatmesseinrichtung zur Analyse des 2. Retentatstroms (in der Beschreibung auch einfach als Messeinrichtung 20b bezeichnet)
- 21a:: 1. Permeatmesseinrichtung zur Analyse des 1. Permeatstroms (in der Beschreibung auch einfach als Messeinrichtung 21a bezeichnet)
- 21b:: 2. Permeatmesseinrichtung zur Analyse des 3. Permeatstroms (in der Beschreibung auch einfach als Messeinrichtung 21b bezeichnet)
- 22:: 3. Permeatmesseinrichtung zur Analyse des 2. Permeatstroms (in der Beschreibung auch einfach als Messeinrichtung 22 bezeichnet)
- 23:: 3. Retentatmesseinrichtung zur Analyse des 3. Retentatstroms (in der Beschreibung auch einfach als Messeinrichtung 23 bezeichnet)
- 24:: Kontrolleinrichtung des Kompressors (in den Abbildungen nicht wiedergegeben)
- 25:: Rohgassteuereinrichtung zur Steuerung des Rohgasstroms (17) in den Abbildungen nicht wiedergegeben)

## Patentansprüche

1. Vorrichtung zur Trennung von Gasen umfassend als Membrantrennstufen zumindest die Feedstromtrennstufe (1), die Retentattrennstufe (2) und die Permeattrennstufe (3) sowie zumindest einen auf der Feedseite der Feedstromtrennstufe (1) angeordneten Kompressor (4) und/oder zumindest eine, bevorzugt eine oder zwei, nach der Feedstromtrennstufe (1), bevorzugt auf der Permeatseite der Retentattrennstufe (2) im zweiten Permeatstrom (9a + 9b) und/oder auf der Permeatseite der Permeattrennstufe (3) im dritten Permeatstrom (11), angeordnete, Vakuumpumpe(n),
wobei
die Feedstromtrennstufe (1) einen Feedstrom (5), bestehend aus mindestens zwei Komponenten, in einen ersten Permeatstrom (6) und einen ersten Retentatstrom (7) auftrennt,
die Retentattrennstufe (2) den ersten Retentatstrom (7) in einen zweiten Permeatstrom (9a + 9b) und einen zweiten Retentatstrom (8), der als Produkt entnommen oder weiterverarbeitet wird, aufteilt und der zweite Permeatstrom dem Feedstrom (5) zugeführt wird,
die Permeattrennstufe (3) den ersten Permeatstrom (6), in einen dritten Retentatstrom (10a + 10b) und einen dritten Permeatstrom (11), der als Produkt entnommen oder weiterverarbeitet oder verworfen wird, aufteilt und der dritte Retentatstrom dem Feedstrom (5) zugeführt wird,
**dadurch gekennzeichnet, dass**
- der zweite Permeatstrom (9a + 9b) mindestens eine Permeatsteuereinrichtung (18) umfasst mit der der Permeatdruck der Retentattrennstufe (2) erhöht oder erniedrigt werden kann und welche anhand von Messwerten eines oder mehrerer Durchflussmesser (20a) im ersten Retentatstrom (7) und/oder eines oder mehrerer Messeinrichtungen (20b) im zweiten Retentatstrom (8) zur Bestimmung der Zusammensetzung des jeweiligen Gasgemisches gesteuert wird und der zweite Permeatstrom (9b) nach der Permeatsteuereinrichtung (18) dem Feedstrom (5) zugeführt wird
und
- der dritte Retentatstrom (10a + 10b) mindestens eine Retentatsteuereinrichtung (19) umfasst mit der der Retentatdruck der Permeattrennstufe (3) erhöht oder erniedrigt werden kann und welche anhand von Messwerten eines oder mehrerer Durchflussmesser (21a) im ersten Permeatstrom (6) und/oder eines oder mehrerer Messeinrichtungen (21b) im dritten Permeatstrom (11) zur Bestimmung der Zusammensetzung des jeweiligen Gasgemisches gesteuert wird.

2. Vorrichtung nach Anspruch 1,
**dadurch gekennzeichnet,**
**dass**
der erste Permeatstrom (6) keiner Rekompression unterworfen wird und/oder
mindestens eine der Membrantrennstufen (1) bis (3) mehr als ein Gasseparationsmembranmodul umfasst, welche parallel und/oder seriell verschaltet sind un
d/oder
das/die Gasseparationsmembranmodul(e) aus Hohlfasermembranen und/oder Flachmembranen bestehen.

3. Vorrichtung nach Anspruch 1 oder 2,
**dadurch gekennzeichnet,**
**dass** der zweite Permeatstrom (9b) und der dritte Retentatstrom (10b) auf die Saugseite des Kompressors (4) geleitet werden
und/oder
**dass** ein mehrstufiger Kompressor (4) verwendet wird, wobei bevorzugt der zweite Permeatstrom (9b) und /oder der dritte Retentatstrom (10b) zwischen zwei Verdichtungsstufen in den Kompressor (4) eingeführt wird/werden und/oder dass der Kompressor (4) in der Vorrichtung derart angeordnet ist, dass er ein Druckgefälle in der Feedstromtrennstufe (1) erzeugt.

4. Vorrichtung nach einem der Ansprüche 1 bis 3,
**dadurch gekennzeichnet,**
**dass** die Vorrichtung eine Kontrolleinrichtung (24) umfasst, welche die Leistung des Kompressors (4), bevorzugt dessen Drehzahl, an Veränderungen des zweiten Permeatstroms (9b) und/oder des dritten Retentatstroms (10b) und/oder des Rohgasstroms (17) anpasst, wobei der Rohgasstrom ein Strom aus mindestens zwei Gasen darstellt, der der erfindungsgemäßen Vorrichtung zugeführt wird um dort aufgetrennt zu werden, und der zusammen mit dem zweiten Permeatstrom (9b) und Retentatstrom (10b) dem Feedstrom (5) zugeführt wird,
und/oder
**dass** die Vorrichtung derart ausgestaltet ist, dass sich ändernde Mengen an rückgeführtem Gas aus dem zweiten Permeatstrom (9b) und/oder dem dritten Retentatstrom (10b), bevorzugt automatisch, mit einer Regelung der zugeführten Menge an Rohgas, bevorzugt über die Rohgassteuereinrichtung (25), ausgeglichen werden, bevorzugt ohne die Drehzahl des Kompressors (4) zu ändern.

5. Vorrichtung nach einem der Ansprüche 1 bis 4,
**dadurch gekennzeichnet,**
**dass** im zweiten Retentatstrom (8) und/oder im dritten Permeatstrom (11) eine online oder offline Messeinrichtung (20b) und/oder (21b) zur Bestimmung der Zusammensetzung des jeweiligen Gasgemisches verwendet wird.

6. Vorrichtung nach einem der Ansprüche 1 bis 5,
**dadurch gekennzeichnet,**
**dass** als Materialien für die trennaktive Schicht der Membranen amorphe oder teilkristalline Kunststoffe verwendet werden wie zum Beispiel aber nicht ausschließlich Polyimide, Polyamide, Polysulfone, Celluloseacetate und Derivate, Polyphenylenoxide, Polysiloxane, Polymere mit intrinsischer Mikroporosität, Mixed Matrix Membranen, Facilitated Transport Membranen, Polyethylenoxide, Polypropylenoxide oder Mischungen daraus.

7. Vorrichtung nach Anspruch 6,
**dadurch gekennzeichnet,**
**dass** als Material für die trennaktive Schicht der Membranen ein Polyimid der allgemeinen Formel 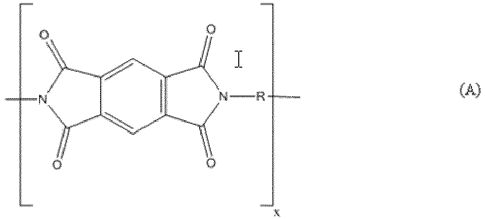 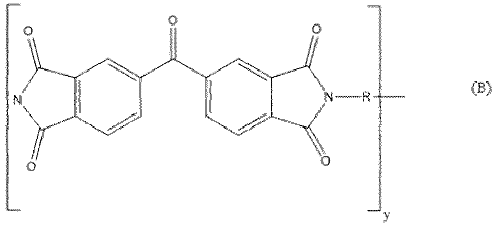 mit 0 ≤ x ≤ 0,5 und 1 ≥ y ≥ 0,5 und R entspricht einem oder mehreren, gleichen oder unterschiedlichen Resten R, ausgewählt aus der Gruppe bestehend aus den Resten L1, L2, L3 und L4 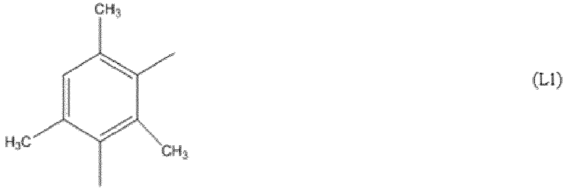 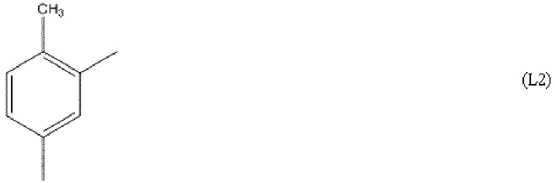 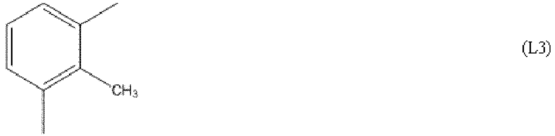 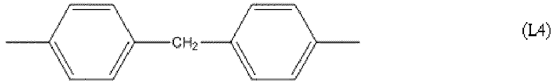 verwendet wird, bevorzugt ein Polyimid mit der CAS-Nr. 9046-51-9 und/oder ein Polyimid mit der CAS-Nr. 134119-41-8.

8. Verfahren zur Steuerung einer Gasseparationsanlage nach einem der Ansprüche 1 bis 7,
**dadurch gekennzeichnet,**
**dass**
i. wenn die Konzentration einer schwerer permeierenden Komponente B, ggf. auch ermittelt durch einen dazu korrelierenden Parameter, des zweiten Retentatstroms (8) unter einen vorgegebenen Sollwert fällt, der Druck des zweiten Permeatstroms (9a) mittels der Permeatsteuereinrichtung (18) erniedrigt wird, bis besagte Konzentration bzw. Parameter wieder im Sollbereich liegt,
ii. wenn die Konzentration einer schwerer permeierenden Komponente B, ggf. auch ermittelt durch einen dazu korrelierenden Parameter, des zweiten Retentatstroms (8) über einen vorgegebenen Sollwert steigt, der Druck des zweiten Permeatstroms (9a) mittels der Permeatsteuereinrichtung (18) erhöht wird, bis besagte Konzentration bzw. Parameter wieder im Sollbereich liegt,
und
iii. wenn die Konzentration einer schwerer permeierenden Komponente B, ggf. auch ermittelt durch einen dazu korrelierenden Parameter, des dritten Permeatstroms (11) unter einen vorgegebenen Sollwert fällt, der Druck des dritten Retentatstroms (10a) mittels der Retentatsteuereinrichtung (19) erhöht wird, bis besagte Konzentration bzw. Parameter wieder im Sollbereich liegt,
iv. wenn die Konzentration einer schwerer permeierenden Komponente B, ggf. auch ermittelt durch einen dazu korrelierenden Parameter, des dritten Permeatstroms (11) über einen vorgegebenen Sollwert steigt, der Druck des dritten Retentatstroms (10a) mittels der Retentatsteuereinrichtung (19) erniedrigt wird, bis besagte Konzentration bzw. Parameter wieder im Sollbereich liegt.

9. Verfahren nach Anspruch 8,
**dadurch gekennzeichnet, dass**
die Ermittlung der Konzentration online und/oder offline erfolgt.

10. Verfahren zur Steuerung einer Gasseparationsanlage nach einem der Ansprüche 1 bis 7,
**dadurch gekennzeichnet,**
**dass**
v. wenn der Volumenstrom des ersten Retentatstroms (7) steigt, der Druck des zweiten Permeatstroms (9a) mittels der Permeatsteuereinrichtung (18) erniedrigt wird, bis eine, anhand einer Kalibrierkurve mit dem Volumenstrom des ersten Retentatstroms (7) korrelierte, Eigenschaft des zweiten Retentatstroms (8), bevorzugt die Zusammensetzung des zweiten Retentatstroms (8), wieder im Sollbereich liegt,
vi. wenn der Volumenstrom des ersten Retentatstroms (7) sinkt, der Druck des zweiten Permeatstroms (9a) mittels der Permeatsteuereinrichtung (18) erhöht wird, bis eine, anhand einer Kalibrierkurve mit dem Volumenstrom des ersten Retentatstroms (7) korrelierte, Eigenschaft des zweiten Retentatstroms (8), bevorzugt die Zusammensetzung des zweiten Retentatstroms (8), wieder im Sollbereich liegt,
und/oder
vii. wenn der Volumenstrom des ersten Permeatstroms (6) steigt, der Druck des dritten Retentatstroms (10a) mittels der Retentatsteuereinrichtung (19) erhöht wird, bis eine, anhand einer Kalibrierkurve mit dem Volumenstrom des ersten Permeatstroms (6) korrelierte, Eigenschaft des dritten Permeatstroms (11), bevorzugt die Zusammensetzung des dritten Permeatstroms (11), wieder im Sollbereich liegt,
viii. wenn der Volumenstrom des ersten Permeatstroms (6) sinkt, der Druck des dritten Retentatstroms (10a) mittels der Retentatsteuereinrichtung (19) erniedrigt wird, bis eine, anhand einer Kalibrierkurve mit dem Volumenstrom des ersten Permeatstroms (6) korrelierte, Eigenschaft des dritten Permeatstroms (11), bevorzugt die Zusammensetzung des dritten Permeatstroms (11), wieder im Sollbereich liegt.

11. Verfahren nach Anspruch 10,
**dadurch gekennzeichnet, dass**
die Regelung nach einer Kalibrierkurve erfolgt, die eine Korrelation zwischen Fluss und Druck enthält zur Aufrechterhaltung einer Konzentration in einem anderen Gasstrom.

12. Verfahren nach einem der Ansprüche 8 bis 11,
**dadurch gekennzeichnet,**
**dass** der Druckabfall über die Feedstromtrennstufe (1) auf 1 bis 30 bar, vorzugsweise auf 2 bis 20 bar und besonders vorzugsweise auf 3 und 10 bar eingestellt wird
und/oder
**dass** der Druckabfall über die Feedstromtrennstufe (1) und die Retentattrennstufe (2) auf 1 bis 100 bar, vorzugsweise auf 5 bis 80 bar und besonders vorzugsweise auf 10 bis 70 bar eingestellt wird.

13. Verfahren nach einem der Ansprüche 8 bis 12,
**dadurch gekennzeichnet,**
**dass** als Triebkraft für die Trennaufgabe eine Partialdruckdifferenz zwischen der Retentat- und der Permeatseite in den jeweiligen Membrantrennstufen zum Einsatz kommt, wobei die Partialdruckdifferenz mittels eines Kompressors (4), welcher auf der Feedseite der Feedstromtrennstufe (1) angeordnet ist, und gegebenenfalls mittels zumindest einer, bevorzugt einer oder zwei Vakuumpumpe(n) im zweiten und/oder dritten Permeatstrom (9a + 9b) und / oder (11) und/oder durch einen permeatseitigen Spülgasstrom erzeugt wird
und/oder
**dass** der Druck des Permeats der Feedstromtrennstufe (1) gleich oder erhöht ist gegenüber dem Umgebungsdruck, sodass noch eine Partialdruckdifferenz zwischen Retentat und Permeat der Permeattrennstufe (3) besteht und damit eine treibende Kraft gegeben ist für den Fall, dass das Permeat der Permeattrennstufe (3) auf Umgebungsdruck ist oder Unterdruck angelegt wird.

14. Verfahren nach einem der Ansprüche 8 bis 13,
**dadurch gekennzeichnet,**
**dass** mittels einer Kontrolleinrichtung (24) die Leistung des Kompressors (4), bevorzugt dessen Drehzahl, an Veränderungen des zweiten Permeatstroms (9b) und/oder des dritten Retentatstroms (10b) und/oder des Rohgasstroms (17) anpasst wird
oder
**dass** sich ändernde Mengen an rückgeführtem Gas aus dem zweiten Permeatstrom (9b) und/oder dem dritten Retentatstrom (10b), bevorzugt automatisch, mit einer Regelung der zugeführten Menge an Rohgas, bevorzugt über die Rohgassteuereinrichtung (25), ausgeglichen werden, bevorzugt ohne die Drehzahl des Kompressors zu ändern
oder
**dass** die Anlagenleistung der erfindungsgemäßen Anlage durch eine Änderung des Fördervolumens des Kompressors (4) erhöht oder erniedrigt wird, wobei
einer dadurch verursachten Änderung der Konzentration der schwerer permeierenden Komponente B im zweiten Retentatstrom (8) gemäß den Verfahrensalternativen i bzw. ii
und/oder
einer dadurch verursachten Änderung der Konzentration der schwerer permeierenden Komponente B im dritten Permeatstrom (11) gemäß den Verfahrensalternativen iii bzw. iv
und/oder
einer dadurch verursachten Änderung des Flusses des ersten Retentatstroms (7) gemäß den Verfahrensalternativen v bzw. vi und/oder
einer dadurch verursachten Änderung des Flusses des ersten Permeatstroms (6) gemäß den Verfahrensalternativen vii bzw. viii entgegengewirkt wird.

15. Verfahren nach einem der Ansprüche 8 bis 14, **dadurch gekennzeichnet,**
**dass** es im Rahmen des Betriebs einer Biogasanlage durchgeführt wird und dass je nach Füllstand der Biogasanlage, bevorzugt ermittelt über Druck des Fermenters oder den Füllstand eines Zwischenspeichers, die Drehzahl des Kompressors und damit das Fördervolumen des Kompressors (4) gesteuert wird, um den Füllstand im Fermenter und/oder Zwischenspeicher zu verändern oder konstant zu halten
und/oder
**dass** als Gasgemisch eine Mischung von vorwiegend aber nicht ausschließlich Kohlendioxid und Methan oder vorwiegend aber nicht ausschließlich Wasserstoff und Methan oder vorwiegend aber nicht ausschließlich Kohlenmonooxid und Wasserstoff oder Rohbiogas oder Roherdgas verwendet wird.

16. Verfahren nach einem der Ansprüche 8 bis 15, **dadurch gekennzeichnet,**
**dass** zumindest in der Feedstromtrennstufe (1), bevorzugt jedoch in allen drei Membrantrennstufen (1) bis (3), Gasseparationsmembranmodule mit einer Gemischtgasselektivität CO₂/CH₄ von mindestens 30, bevorzugt mindestens 35, besonders bevorzugt mindestens 40 und ganz besonders bevorzugt mindestens 45 verwendet werden
und/oder
**dass** das im zweiten Permeatstrom (9b) und im dritten Retentatstrom (10b) rückgeführte Gasvolumen in Summe weniger als 60 Vol. % des Volumens des Rohgasstroms (17) beträgt,
und/oder
**dass** die Konzentration mindestens eines Permeatgases der Feedstromtrennstufe (1), nach Rückführung des zweiten Permeatstroms (9b) und des dritten Retentatstroms (10b), im Feedstrom (5) erhöht wird, bevorzugt um mindestens 2%, besonders bevorzugt um mindestens 3% und besonders bevorzugt um 3 bis 40%, jeweils im Vergleich zur Konzentration im Rohgasstrom (17).

17. Biogasanlage umfassend eine Vorrichtung nach einem der Ansprüche 1 bis 7.

## Claims

1. Apparatus for separating gases, comprising as membrane separation stages at least a feed stream separation stage (1), a retentate separation stage (2) and a permeate separation stage (3) and also at least one compressor (4) arranged on the feed side of said feed stream separation stage (1) and/or at least one, preferably one or two, vacuum pump(s) arranged downstream of said feed stream separation stage (1), preferably on the permeate side of said retentate separation stage (2) in the second permeate stream (9a + 9b) and/or on the permeate side of said permeate separation stage (3) in the third permeate stream (11),
wherein
said feed stream separation stage (1) separates a feed stream (5), consisting of two or more components, into a first permeate stream (6) and a first retentate stream (7),
said retentate separation stage (2) divides said first retentate stream (7) into a second permeate stream (9a + 9b) and a second retentate stream (8) which is removed as product or further processed, and said second permeate stream is supplied to said feed stream (5),
said permeate separation stage (3) divides said first permeate stream (6) into a third retentate stream (10a + 10b) and a third permeate stream (11), which is removed as product or further processed or discarded, and said third retentate stream is supplied to said feed stream (5), **characterized in that**
- said second permeate stream (9a + 9b) comprises at least one permeate control means (18) with which the permeate pressure of said retentate separation stage (2) can be raised or lowered and which is controlled on the basis of measured values from one or more flow meters (20a) in said first retentate stream (7) and/or one or more measuring means (20b) in said second retentate stream (8) for determining the composition of the particular gas mixture and said second permeate stream (9b) is supplied to said feed stream (5) downstream of said permeate control means (18),
and
- said third retentate stream (10a + 10b) comprises at least one retentate control means (19) with which the retentate pressure of said permeate separation stage (3) can be raised or lowered and which is controlled on the basis of measured values from one or more flow meters (21a) in said first permeate stream (6) and/or one or more measuring means (21b) in said third permeate stream (11) for determining the composition of the particular gas mixture.

2. Apparatus according to Claim 1,
**characterized in that**
said first permeate stream (6) is not subjected to recompression
and/or
at least one of said membrane separation stages (1) to (3) comprises more than one gas separation membrane module interconnected in parallel and/or in series,
and/or
the gas separation membrane module(s) consist(s) of hollow fibre membranes and/or flat membranes.

3. Apparatus according to Claim 1 or 2, char
acterized in that
said second permeate stream (9b) and said third retentate stream (10b) are led to the suction side of said compressor (4),
and/or in that
a multi-stage compressor (4) is used, wherein preferably said second permeate stream (9b) and/or said third retentate stream (10b) is/are introduced into said compressor (4) between two compression stages,
and/or in that said compressor (4) is arranged in said apparatus such that it generates a pressure gradient in said feed stream separation stage (1).

4. Apparatus according to any of Claims 1 to 3, cha
racterized in that
said apparatus comprises a controller means (24) which adapts the capacity of said compressor (4), preferably its rotary speed, to changes in said second permeate stream (9b) and/or said third retentate stream (10b) and/or said raw gas stream (17), wherein said raw gas stream is a stream of at least two gases which is supplied to the apparatus according to the invention in order to be separated therein and which together with said second permeate stream (9b) and retentate stream (10b) is supplied to said feed stream (5), and
/or in that
said apparatus is configured such that changing amounts of recycled gas from said second permeate stream (9b) and/or said third retentate stream (10b) are compensated, preferably automatically, by a regulation of the supplied amount of raw gas, preferably via a raw gas control means (25), preferably without changing the rotary speed of said compressor (4).

5. Apparatus according to any of Claims 1 to 4, **characterized in that**
an online or offline measuring means (20b) and/or (21b) is used in said second retentate stream (8) and/or in said third permeate stream (11) to determine the composition of the particular gas mixture.

6. An apparatus according to any of Claims 1 to 5, **characterized in that**
the materials used for the separation-active layer of the membranes are amorphous or partly crystalline plastics such as, for example but not exclusively, polyimides, polyamides, polysulphones, cellulose acetates and derivatives, polyphenylene oxides, polysiloxanes, polymers having intrinsic microporosity, mixed matrix membranes, facilitated transport membranes, polyethylene oxides, polypropylene oxides or mixtures thereof.

7. Apparatus according to Claim 6,
**characterized in that**
the material used for the separation-active layer of the membranes is a polyimide of the general formula 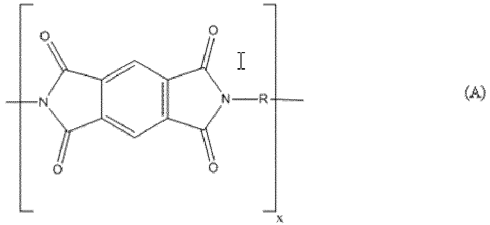 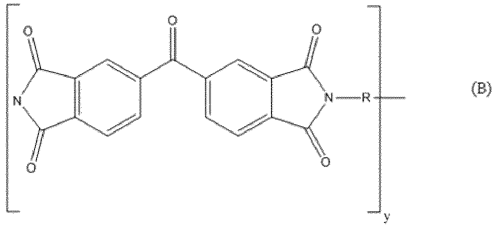 where 0 ≤ x ≤ 0.5 and 1 ≥ y ≥ 0.5 and R corresponds to one or more, identical or different radicals R selected from the group consisting of the radicals L1, L2, L3 and L4, 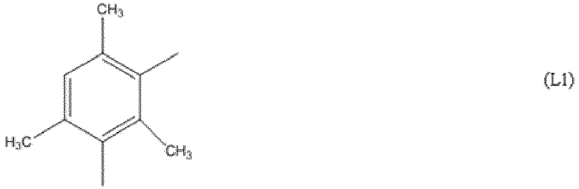 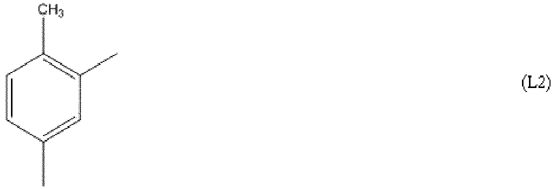 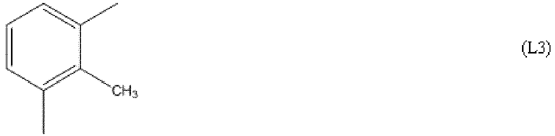 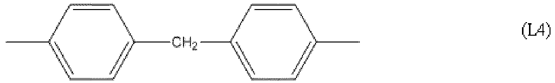 preferably a polyimide of CAS number 9046-51-9 and/or a polyimide of CAS number 134119-41-8.

8. Method of controlling a gas separation plant according to any of Claims 1 to 7, **characterized in that**
i. when the concentration of a less readily permeating component B, optionally also determined from a parameter correlating therewith, of said second retentate stream (8) falls below a predetermined setpoint value, the pressure of said second permeate stream (9a) is lowered by said permeate control means (18) until said concentration/parameter is back in the setpoint range,
ii. when the concentration of a less readily permeating component B, optionally also determined from a parameter correlating therewith, of said second retentate stream (8) rises above a predetermined setpoint value, the pressure of said second permeate stream (9a) is raised by said permeate control means (18) until said concentration/parameter is back in the setpoint range,
and
iii. when the concentration of a less readily permeating component B, optionally also determined from a parameter correlating therewith, of said third permeate stream (11) falls below a predetermined setpoint value, the pressure of said third retentate stream (10a) is raised by said retentate control means (19) until said concentration/parameter is back in the setpoint range,
iv. when the concentration of a less readily permeating component B, optionally also determined from a parameter correlating therewith, of said third permeate stream (11) rises above a predetermined setpoint value, the pressure of said third retentate stream (10a) is lowered by said retentate control means (19) until said concentration/parameter is back in the setpoint range.

9. Method according to Claim 8,
**characterized in that**
the concentration is determined online and/or offline.

10. Method of controlling a gas separation plant according to any of Claims 1 to 7,
**characterized in that**
v. when the volume flow of said first retentate stream (7) increases, the pressure of said second permeate stream (9a) is lowered by said permeate control means (18) until a property of said second retentate stream (8) which is correlated by a calibration curve with said volume flow of said first retentate stream (7), preferably the composition of said second retentate stream (8), is back in the setpoint range,
vi. when the volume flow of said first retentate stream (7) decreases, the pressure of said second permeate stream (9a) is raised by said permeate control means (18) until a property of said second retentate stream (8) which is correlated by a calibration curve with said volume flow of said first retentate stream (7), preferably the composition of said second retentate stream (8), is back in the setpoint range,
and/or
vii. when the volume flow of said first permeate stream (6) increases, the pressure of said third retentate stream (10a) is raised by said retentate control means (19) until a property of said third permeate stream (11) which is correlated by a calibration curve with said volume flow of said first permeate stream (6), preferably the composition of said third permeate stream (11), is back in the setpoint range,
viii. when the volume flow of said first permeate stream (6) decreases, the pressure of said third retentate stream (10a) is lowered by said retentate control means (19) until a property of said third permeate stream (11) which is correlated by a calibration curve with said volume flow of said first permeate stream (6), preferably the composition of said third permeate stream (11), is back in the setpoint range.

11. Method according to Claim 10,
**characterized in that**
a calibration curve containing a correlation between flow rate and pressure is used as control curve to maintain a concentration in some other gas stream.

12. Method according to any of Claims 8 to 11, **characterized in that**
the pressure drop across said feed stream separation stage (1) is set at from 1 to 30 bar, preferably at from 2 to 20 bar and more preferably at 3 and 10 bar,
and/or **in that**
the pressure drop across said feed stream separation stage (1) and said retentate separation stage (2) is set at from 1 to 100 bar, preferably at from 5 to 80 bar and more preferably at from 10 to 70 bar.

13. Method according to any of Claims 8 to 12, char
acterized in that
the driving force used for the separation task is a partial pressure difference between the retentate side and the permeate side in the particular membrane separation stages, wherein said partial pressure difference is generated by a compressor (4), which is arranged on the feed side of said feed stream separation stage (1), and optionally by at least one, preferably one or two vacuum pump(s) in said second and/or third permeate stream (9a + 9b) and/or (11) and/or by a permeate-side purge gas stream,
and/or in that
the pressure of the permeate of said feed stream separation stage (1) is in an equal or elevated state relative to the ambient pressure, so there is still a partial pressure difference between the retentate and the permeate of said permeate separation stage (3) and hence there is a driving force in the event that said permeate of said permeate separation stage (3) is at ambient pressure or negative pressure is applied.

14. Method according to any of Claims 8 to 13, cha
racterized in that
a controller means (24) adapts the capacity of said compressor (4), preferably its rotary speed, to changes in the second permeate stream (9b) and/or said third retentate stream (10b) and/or said raw gas stream (17),
or in that
changing amounts of recycled gas from said second permeate stream (9b) and/or said third retentate stream (10b) are compensated, preferably automatically, by a regulation of the supplied amount of raw gas, preferably via said raw gas control means (25), preferably without changing the rotary speed of said compressor
or in that
the performance of the plant of the present invention is raised or lowered by changing the volume throughput of said compressor (4), wherein
a resultant change in the concentration of said less readily permeating component B in said second retentate stream (8) is counteracted according to method alternatives i/ii,
and/or
a resultant change in the concentration of said less readily permeating component B in said third permeate stream (11) is counteracted according to method alternatives iii/iv
and/or
a resultant change in the flow rate of said first retentate stream (7) is counteracted according to method alternatives v/vi,
and/or
a resultant change in the flow rate of said first permeate stream (6) is counteracted according to method alternatives vii/viii.

15. Method according to any of Claims 8 to 14, **characterized in that**
said method is practised in the context of operating a biogas plant and **in that** the rotary speed of the compressor and hence the volume throughput of said compressor (4) is controlled according to said biogas plant fill level, preferably determined via fermenter pressure or intermediate store fill level, in order that the fill level in the fermenter and/or intermediate store may be changed or kept constant,
and/or **in that**
the gas mixture used is a mixture of predominantly but not exclusively carbon dioxide and methane or predominantly but not exclusively hydrogen and methane or predominantly but not exclusively carbon monoxide and hydrogen or raw biogas or raw natural gas.

16. Method according to any of Claims 8 to 15, **characterized in that**
gas separation membrane modules having a mixed gas selectivity CO₂/CH₄ of not less than 30, preferably not less than 35, more preferably not less than 40 and most preferably not less than 45 are used at least in said feed stream separation stage (1), but preferably in all three membrane separation stages (1) to (3),
and/or **in that**
the gas volume recycled in said second permeate stream (9b) and in said third retentate stream (10b) amounts in total to less than 60% by volume of the volume of the raw gas stream (17),
and/or **in that**
the concentration of at least one permeate gas of said feed stream separation stage (1), after returning said second permeate stream (9b) and said third retentate stream (10b), is raised in said feed stream (5), preferably by not less than 2%, more preferably by not less than 3% and even more preferably by 3 to 40%, all compared with the concentration in said raw gas stream (17).

17. Biogas plant comprising an apparatus according to any of Claims 1 to 7.

## Revendications

1. Dispositif pour la séparation de gaz comprenant en tant qu'étapes de séparation sur membrane au moins l'étape de séparation du courant d'alimentation (1), l'étape de séparation du rétentat (2) et l'étape de séparation du perméat (3), ainsi qu'au moins un compresseur (4) agencé sur le côté de l'alimentation de l'étape de séparation du courant d'alimentation (1) et/ou au moins une, de préférence une ou deux, pompes à vide agencées après l'étape de séparation du courant d'alimentation (1), de préférence sur le côté du perméat de l'étape de séparation du rétentat (2) dans le deuxième courant de perméat (9a + 9b) et/ou sur le côté du perméat de l'étape de séparation du perméat (3) dans le troisième courant de perméat (11),
l'étape de séparation du courant d'alimentation (1) séparant un courant d'alimentation (5), constitué par au moins deux composants, en un premier courant de perméat (6) et un premier courant de rétentat (7),
l'étape de séparation du rétentat (2) séparant le premier courant de rétentat (7) en un deuxième courant de perméat (9a + 9b) et un deuxième courant de rétentat (8), qui est soutiré en tant que produit ou transformé, et le deuxième courant de perméat étant introduit dans le courant d'alimentation (5),
l'étape de séparation du perméat (3) séparant le premier courant de perméat (6) en un troisième courant de rétentat (10a + 10b) et un troisième courant de perméat (11), qui est soutiré en tant que produit ou transformé ou mis au rebut, et le troisième courant de rétentat étant introduit dans le courant d'alimentation (5), **caractérisé en ce que**
- le deuxième courant de perméat (9a + 9b) comprend au moins un dispositif de régulation du perméat (18) avec lequel la pression du perméat de l'étape de séparation du rétentat (2) peut être augmentée ou réduite, et qui est régulé à partir de valeurs de mesure d'un ou de plusieurs débitmètres (20a) dans le premier courant de rétentat (7) et/ou d'un ou de plusieurs dispositifs de mesure (20b) dans le deuxième courant de rétentat (8) pour la détermination de la composition du mélange gazeux en question, et le deuxième courant de perméat (9b) est introduit dans le courant d'alimentation (5) après le dispositif de régulation du perméat (18),
et
- le troisième courant de rétentat (10a + 10b) comprend au moins un dispositif de régulation du rétentat (19) avec lequel la pression du rétentat de l'étape de séparation du perméat (3) peut être augmentée ou réduite, et qui est régulé à partir de valeurs de mesure d'un ou de plusieurs débitmètres (21a) dans le premier courant de perméat (6) et/ou d'un ou de plusieurs dispositifs de mesure (21b) dans le troisième courant de perméat (11) pour la détermination de la composition du mélange gazeux en question.

2. Dispositif selon la revendication 1, **caractérisé en ce que**
le premier courant de perméat (6) n'est pas soumis à une recompression et/ou
au moins une des étapes de séparation sur membrane (1) à (3) comprend plus d'un module à membrane de séparation de gaz, qui sont raccordés en parallèle et/ou en série, et/ou
le ou les modules à membrane de séparation de gaz sont constitués par des membranes fibres creuses et/ou des membranes plates.

3. Dispositif selon la revendication 1 ou 2, **caractérisé en ce que** le deuxième courant de perméat (9b) et/ou le troisième courant de rétentat (10b) sont conduits sur le côté de l'aspiration du compresseur (4), et/ou
**en ce qu'**un compresseur à plusieurs étages (4) est utilisé, le deuxième courant de perméat (9b) et/ou le troisième courant de rétentat (10b) étant de préférence introduits dans le compresseur (4) entre deux étages de compression,
et/ou **en ce que** le compresseur (4) est agencé dans le dispositif de telle sorte qu'il génère une différence de pression dans l'étape de séparation du courant d'alimentation (1).

4. Dispositif selon l'une quelconque des revendications 1 à 3, **caractérisé en ce que** le dispositif comprend un dispositif de contrôle (24), qui adapte la puissance du compresseur (4), de préférence sa vitesse de rotation, à des modifications du deuxième courant de perméat (9b) et/ou du troisième courant de rétentat (10b) et/ou du courant de gaz brut (17), le courant de gaz brut étant un courant constitué par au moins deux gaz qui est introduit dans le dispositif selon l'invention pour y être séparé, et qui est introduit conjointement avec le deuxième courant de perméat (9b) et le courant de rétentat (10b) dans le courant d'alimentation (5),
et/ou
**en ce que** le dispositif est configuré de telle sorte que des quantités variables de gaz recyclé à partir du deuxième courant de perméat (9b) et/ou du troisième courant de rétentat (10b) soient compensées, de préférence automatiquement, par une régulation de la quantité introduite de gaz brut, de préférence par le dispositif de régulation du gaz brut (25), de préférence sans modifier la vitesse de rotation du compresseur (4).

5. Dispositif selon l'une quelconque des revendications 1 à 4, **caractérisé en ce qu'**un dispositif de mesure en ligne ou hors ligne (20b) et/ou (21b) pour la détermination de la composition du mélange gazeux en question est utilisé dans le deuxième courant de rétentat (8) et/ou dans le troisième courant de perméat (11).

6. Dispositif selon l'une quelconque des revendications 1 à 5, **caractérisé en ce que** des plastiques amorphes ou partiellement cristallins sont utilisés en tant que matériaux pour la couche à activité de séparation des membranes, tels que par exemple, mais toutefois pas exclusivement, des polyimides, des polyamides, des polysulfones, des acétates de cellulose et des dérivés, des polyoxydes de phénylène, des polysiloxanes, des polymères à microporosité intrinsèque, des membranes à matrice mixte, des membranes à transport facilité, des polyoxydes d'éthylène, des polyoxydes de propylène ou leurs mélanges.

7. Dispositif selon la revendication 6, **caractérisé en ce qu'**un polyimide de formule générale 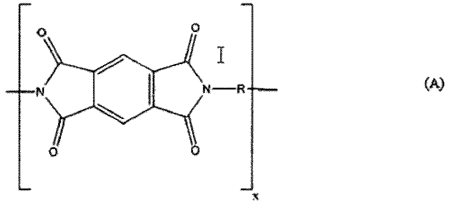 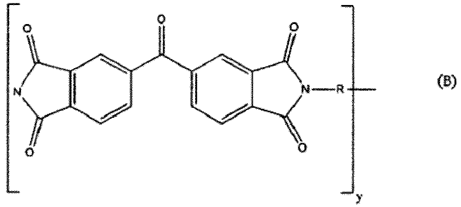 est utilisé en tant que matériau pour la couche à activité de séparation des membranes,
avec 0 ≤ x ≤ 0,5 et 1 ≥ y ≥ 0,5, et R correspondant à un ou plusieurs radicaux R identiques ou différents, choisis dans le groupe constitué par les radicaux L1, L2, L3 et L4 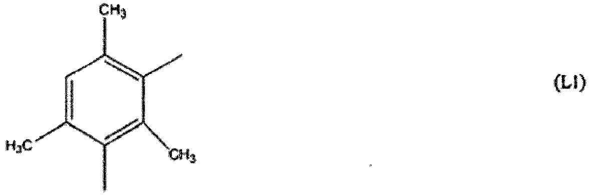 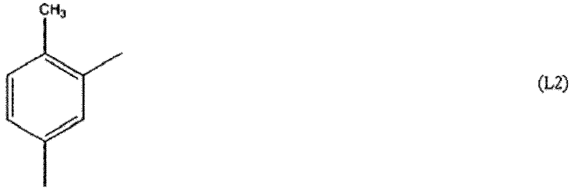 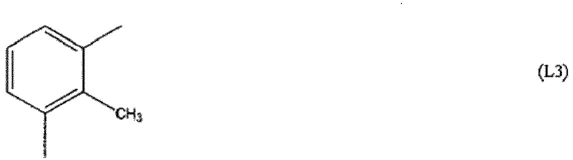 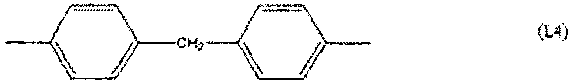 de préférence un polyimide de n° CAS 9046-51-9 et/ou un polyimide de n° CAS 134119-41-8.

8. Procédé de régulation d'une unité de séparation de gaz selon l'une quelconque des revendications 1 à 7, **caractérisé en ce que**
i. lorsque la concentration d'un composant B perméant plus difficilement, éventuellement également déterminée par un paramètre corrélé à celle-ci, du deuxième courant de rétentat (8) chute en dessous d'une valeur de consigne prédéterminée, la pression du deuxième courant de perméat (9a) est réduite au moyen du dispositif de régulation du perméat (18) jusqu'à ce que ladite concentration ou ledit paramètre se situe de nouveau dans la plage de consigne,
ii. lorsque la concentration d'un composant B perméant plus difficilement, éventuellement également déterminée par un paramètre corrélé à celle-ci, du deuxième courant de rétentat (8) augmente au-dessus d'une valeur de consigne prédéterminée, la pression du deuxième courant de perméat (9a) est augmentée au moyen du dispositif de régulation du perméat (18) jusqu'à ce que ladite concentration ou ledit paramètre se situe de nouveau dans la plage de consigne,
et
iii. lorsque la concentration d'un composant B perméant plus difficilement, éventuellement également déterminée par un paramètre corrélé à celle-ci, du troisième courant de perméat (11) chute en dessous d'une valeur de consigne prédéterminée, la pression du troisième courant de rétentat (10a) est augmentée au moyen du dispositif de régulation du rétentat (19) jusqu'à ce que ladite concentration ou ledit paramètre se situe de nouveau dans la plage de consigne,
iv. lorsque la concentration d'un composant B perméant plus difficilement, éventuellement également déterminée par un paramètre corrélé à celle-ci, du troisième courant de perméat (11) augmente au-dessus d'une valeur de consigne prédéterminée, la pression du troisième courant de rétentat (10a) est réduite au moyen du dispositif de régulation du rétentat (19) jusqu'à ce que ladite concentration ou ledit paramètre se situe de nouveau dans la plage de consigne.

9. Procédé selon la revendication 8, **caractérisé en ce que** la détermination de la concentration a lieu en ligne et/ou hors ligne.

10. Procédé de régulation d'une unité de séparation de gaz selon l'une quelconque des revendications 1 à 7, **caractérisé en ce que**
v. lorsque le débit volumique du premier courant de rétentat (7) augmente, la pression du deuxième courant de perméat (9a) est réduite au moyen du dispositif de régulation du perméat (18) jusqu'à ce qu'une propriété du deuxième courant de rétentat (8), de préférence la composition du deuxième courant de rétentat (8), corrélée avec le débit volumique du premier courant de rétentat (7) à l'aide d'une courbe de calibration, se situe de nouveau dans la plage de consigne,
vi. lorsque le débit volumique du premier courant de rétentat (7) diminue, la pression du deuxième courant de perméat (9a) est augmentée au moyen du dispositif de régulation du perméat (18) jusqu'à ce qu'une propriété du deuxième courant de rétentat (8), de préférence la composition du deuxième courant de rétentat (8), corrélée avec le débit volumique du premier courant de rétentat (7) à l'aide d'une courbe de calibration, se situe de nouveau dans la plage de consigne,
et/ou
vii. lorsque le débit volumique du premier courant de perméat (6) augmente, la pression du troisième courant de rétentat (10a) est augmentée au moyen du dispositif de régulation du rétentat (19) jusqu'à ce qu'une propriété du troisième courant de perméat (11), de préférence la composition du troisième courant de perméat (11), corrélée avec le débit volumique du premier courant de perméat (6) à l'aide d'une courbe de calibration, se situe de nouveau dans la plage de consigne,
viii. lorsque le débit volumique du premier courant de perméat (6) diminue, la pression du troisième courant de rétentat (10a) est réduite au moyen du dispositif de régulation du rétentat (19) jusqu'à ce qu'une propriété du troisième courant de perméat (11), de préférence la composition du troisième courant de perméat (11), corrélée avec le débit volumique du premier courant de perméat (6) à l'aide d'une courbe de calibration, se situe de nouveau dans la plage de consigne.

11. Procédé selon la revendication 10, **caractérisé en ce que** la régulation a lieu selon une courbe de calibration, qui contient une corrélation entre le débit et la pression pour le maintien d'une concentration dans un autre courant gazeux.

12. Procédé selon l'une quelconque des revendications 8 à 11, **caractérisé en ce que** la chute de pression dans l'étape de séparation du courant d'alimentation (1) est ajustée de 1 à 30 bar, de préférence de 2 à 20 bar et de manière particulièrement préférée de 3 à 10 bar,
et/ou
**en ce que** la chute de pression dans l'étape de séparation du courant d'alimentation (1) et l'étape de séparation du rétentat (2) est ajustée de 1 à 100 bar, de préférence de 5 à 80 bar et de manière particulièrement préférée de 10 à 70 bar.

13. Procédé selon l'une quelconque des revendications 8 à 12, **caractérisé en ce qu'**une différence de pression partielle entre le côté du rétentat et du perméat dans les étapes de séparation sur membrane respectives est utilisée en tant que force motrice pour la séparation, la différence de pression partielle étant générée au moyen d'un compresseur (4), qui est agencé sur le côté de l'alimentation de l'étape de séparation du courant d'alimentation (1), et éventuellement au moyen d'au moins une, de préférence d'une ou de deux pompes à vide dans le deuxième et/ou le troisième courant de perméat (9a + 9b) et/ou (11) et/ou par un courant de gaz de rinçage du côté du perméat,
et/ou
**en ce que** la pression du perméat de l'étape de séparation du courant d'alimentation (1) est identique ou augmentée par rapport à la pression ambiante, de telle sorte qu'une différence de pression partielle existe encore entre le rétentat et le perméat de l'étape de séparation du perméat (3), et qu'une force motrice soit par conséquent présente dans le cas où le perméat de l'étape de séparation du perméat (3) est à la pression ambiante ou une sous-pression est appliquée.

14. Procédé selon l'une quelconque des revendications 8 à 13, **caractérisé en ce qu'**au moyen d'un dispositif de contrôle (24), la puissance du compresseur (4), de préférence sa vitesse de rotation, est adaptée à des modifications du deuxième courant de perméat (9b) et/ou du troisième courant de rétentat (10b) et/ou du courant de gaz brut (17),
ou
**en ce que** des quantités variables de gaz recyclé à partir du deuxième courant de perméat (9b) et/ou du troisième courant de rétentat (10b) sont compensées, de préférence automatiquement, par une régulation de la quantité introduite de gaz brut, de préférence par le dispositif de régulation du gaz brut (25), de préférence sans modifier la vitesse de rotation du compresseur,
ou
**en ce que** la puissance de l'unité selon l'invention est augmentée ou réduite par une modification du volume transporté par le compresseur (4),
une modification ainsi provoquée de la concentration du composant B, perméant plus difficilement, dans le deuxième courant de rétentat (8) étant contrée selon les variantes de procédé i ou ii
et/ou
une modification ainsi provoquée de la concentration du composant B, perméant plus difficilement, dans le troisième courant de perméat (11) étant contrée selon les variantes de procédé iii ou iv
et/ou
une modification ainsi provoquée du débit du premier courant de rétentat (7) étant contrée selon les variantes de procédé v ou vi
et/ou
une modification ainsi provoquée du débit du premier courant de perméat (6) étant contrée selon les variantes de procédé vii ou viii.

15. Procédé selon l'une quelconque des revendications 8 à 14, **caractérisé en ce qu'**il est réalisé dans le cadre de l'exploitation d'une unité de production de biogaz et **en ce que**, selon le niveau de remplissage de l'unité de production de biogaz, de préférence déterminé par la pression du fermentateur ou le niveau de remplissage d'un dispositif de stockage intermédiaire, la vitesse de rotation du compresseur, et ainsi le volume transporté par le compresseur (4), est régulée afin de modifier ou de maintenir constant le niveau de remplissage dans le fermentateur et/ou le dispositif de stockage intermédiaire,
et/ou
**en ce qu'**un mélange prédominamment, mais toutefois pas exclusivement, de dioxyde de carbone et de méthane, ou prédominamment, mais toutefois pas exclusivement, d'hydrogène et de méthane, ou prédominamment, mais toutefois pas exclusivement, de monoxyde de carbone et d'hydrogène, ou un biogaz brut ou un gaz naturel brut est utilisé en tant que mélange gazeux.

16. Procédé selon l'une quelconque des revendications 8 à 15, **caractérisé en ce qu'**au moins dans l'étape de séparation du courant d'alimentation (1), de préférence toutefois dans les trois étapes de séparation sur membrane (1) à (3), des modules à membrane de séparation de gaz ayant une sélectivité du gaz de mélange CO₂/CH₄ d'au moins 30, de préférence d'au moins 35, de manière particulièrement préférée d'au moins 40 et de manière tout particulièrement préférée d'au moins 45, sont utilisés,
et/ou
**en ce que** le volume de gaz recyclé dans le deuxième courant de perméat (9b) et dans le troisième courant de rétentat (10b) est au total de moins de 60 % en volume du volume du courant de gaz brut (17),
et/ou
**en ce que** la concentration d'au moins un gaz de perméat de l'étape de séparation du courant d'alimentation (1) est augmentée après le recyclage du deuxième courant de perméat (9b) et du troisième courant de rétentat (10b), dans le courant d'alimentation (5), de préférence d'au moins 2 %, de manière particulièrement préférée d'au moins 3 % et de manière particulièrement préférée de 3 à 40 %, à chaque fois en comparaison de la concentration dans le courant de gaz brut (17).

17. Unité de production de biogaz, comprenant un dispositif selon l'une quelconque des revendications 1 à 7.
